(19) Europäisches Patentamt
European Patent Office
Office européen des brevets

(11) EP 1 331 219 A1

(12) **EUROPEAN PATENT APPLICATION**

(43) Date of publication:
**30.07.2003 Bulletin 2003/31**

(21) Application number: **02290169.8**

(22) Date of filing: **23.01.2002**

(51) Int Cl.[7]: **C07C 229/14**, C07C 217/60, C07C 323/60, C07K 16/44, A61K 39/00, A61K 39/385, A61K 39/395, C12N 5/20, C12N 5/10, C12N 15/79

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Drug Abuse Sciences, Inc.
Hayward, California 94545-3816 (US)**

(72) Inventors:
- **Pouletty, Philippe
75005 Paris (FR)**
- **Kusmierek, Jacques
75015 Paris (FR)**
- **Koralewski, Frédéric
50340 Les Pieux (FR)**
- **Galons, Hervé
75014 Paris (FR)**
- **Blanchard, Dominique
44300 Nantes (FR)**
- **Gadjou, Caroline, c/o Madame Kamtchoum
94310 Orly-Ville (FR)**
- **Danger, Yannic
44000 Nantes (FR)**

(74) Representative: **Breesé, Pierre
BREESE - MAJEROWICZ,
3, avenue de l'Opéra
75001 Paris (FR)**

(54) **New amphetamine derivatives, antibodies against them and pharmaceutical compositions containing them**

(57) Hapten-carrier conjugates capable of eliciting anti-hapten antibodies in vivo to amphetamines are disclosed. Methods of preparing the hapten-carrier conjugates and therapeutic compositions are also disclosed. A therapeutic composition containing the hapten-carrier conjugate is useful in the treatment of addiction to amphetamines. Passive immunization using antibodies raised against conjugates of the instant invention also is disclosed. The therapeutic composition is suitable for co-therapy with other conventional drugs for treatment of amphetamine abuse.

EP 1 331 219 A1

## Description

### Technical Field

The present invention relates to methods and compositions for treatment of amphetamine abuse using either amphetamine-hapten conjugates directly to elicit antibody production in an abuser or indirectly by using them to prepare therapeutic antibodies which can be used for treatment. The invention is exemplified by preparation of antibodies that react with two or more amphetamine derivatives, including ecstasy as one of the derivatives.

### Background

The abuse of amphetamines is steadily increasing (Karch, et al. J Forensic Sci 1999; 44:359-368). Several products are used including methamphetamine (MA), 3,4-methylenedioxymethamphetamine (MDMA; ecstasy); and 3,4-methylene-dioxyethamphetamine (MDEA, "eve"), but new products continue to appear, most of which exhibit both acute and chronic toxicity (Elliott SP. J Anal Toxicol 2000 Mar;24:85-89; Portion AJ; Lock E. Forensic Sci Int 1999;100: 221-233; Felgate, et al., J Anal Toxicol 1998, 22: 169-172). However, methamphetamine is used considerably less often than ecstasy. Furthermore other derivatives are also used and mixtures of amphetamines are frequently found in the formulations that are abused. In addition pharmacokinetic interactions with some other products can lead to an enhanced toxicity of the abused substance(s).

At present there is no specific treatment for intoxication with amphetamine derivatives. It therefore is of interest to develop monoclonal and polyclonal antibodies that display a sufficient cross reactivity against the main amphetamines of abuse and, in particular, against MDMA ecstasy, particularly neutralizing antibodies against methamphetamine, ecstasy and other amphetamine derivatives. These broad specific antibodies would be of significant therapeutic value in the emergency treatment of drug abuse.

### Relevant Literature

Antibodies against amphetamines have been produced using several different antigens. *See* for example Burgess C, et al.; Eur Psychiatry 2000;15(5):287-294; 5,976,812: and Huber et al; USPN 5,470,997. The antibodies described generally are directed against amphetamine and methamphetamine (see for example, USPN 5,135,863: Hiramatsu M, et al., Pharmacol Biochem Behav 1989; 33:343-347; K.A.A Bymes-Blake, et al., International Immunopharmacology 1, 2001, 329-338; S. Inayama, et al., Chem Pharm Bull 28, 1980, 2779; S. Inayama, et al., Chem Pharm Bull 1977 35 838; K. Aoki, Y. Kuroiwa, J. Pharm Dyn 1983, 6, 33-38). In most cases the antibodies obtained are highly specific antibodies for a single amphetamine and are used for analytical purposes either to detect the presence of a particular amphetamine and/or to distinguish among different amphetamine derivatives.

### SUMMARY

Novel compositions based on amphetamine and derivatives of amphetamine, and their use for treatment of substance abuse are provided. The compositions include new amphetamine derivatives as well as immunoglobulins or immunoglobulin fragments or chains which specifically bind to at least two analogs of amphetamine, one of which is ecstasy, 4-methylthio-amphetamine, metamphetamine, or N-ethylamphetamine. For polyclonal and monoclonal antibody production, animals are immunized with antigens comprising linking groups bonded to the aromatic group or to a side chain of the amphetamine derivative for conjugation to a carrier protein. B cells from an immunized host can be used to prepare hybridomas which produce monoclonal antibodies to amphetamine and derivatives of amphetamine or nucleic acid encoding heavy and/or light chains of the monoclonal antibodies can be isolated and used to prepare recombinant immunoglobulins, fragments or chains in a host cell, which may be prokaryotic or eukaryotic . The polyclonal and monoclonal antibodies find use in detection assays for amphetamine and its analogs, for treatment of an overdose of an amphetamine, and in amphetamine detoxification, as well as prevention of abuse and/or overdose via immunization with an amphetamine derivative-protein conjugate.

### BRIEF DESCRIPTION OF THE DRAWINGS

Figure 1 shows the synthesis of compounds of formula II.
Figure 2 shows synthesis of compounds of formula IIIa and IIIb.
Figure 3 shows an alternative synthesis of compounds of formula IIIb.

Figure 4 shows results from cross reactivity analyses of murine monoclonal antibodies raised against Met1 with human plasma proteins (gel 10%).

BRIEF DESCRIPTION OF THE SPECIFIC EMBODIMENTS

In accordance with the subject invention compounds are provided which are derivatives of amphetamine. The compounds find use as intermediates for the production of immunogens or as the immunogens when coupled to an antigenic protein, such as tetanus toxoid toxin or keyhole limpet hemocyanin (KLH) via either the aromatic ring or a side chain of the amphetamine derivatives. Preferably the linking groups are bound to the aromatic group or to a side chain of the amphetamine derivative at a position so that antibodies are produced against amphetamines substituted on the aromatic ring. Animals, including humans for treatment and/or inhibition of the effects of drug abuse, are immunized for the production of antisera containing antibodies which specifically bind to analogs of amphetamine, preferably at least two analogs, including ecstasy, 4-methylthioamphetamine, metamphetamine, or N-ethylamphetamine. B cells from the immunized animal host can be used to produce hybridomas which produce monoclonal antibodies having the same specificity spectrum. Recombinant immunoglobulin light chain and/or heavy chains and functional fragments thereof can be made recombinantly by isolating nucleic acid encoding the monoclonal antibody light and heavy chains or functional portions thereof and expressing it in a prokaryotic host cell such as E. coli or a eukaryotic host cell such as a yeast or mammalian cell. The recombinant antibodies can include alterations in the amino acid sequence to provide for desired characteristics, for example changes can be made in the variable region to provide improved antigen binding characteristics. Hybrid antibodies also can be prepared that recognize two antigens simultaneously. By "hybrid antibodies" is intended antibodies in which one pair of heavy and light chains is homologous to antibodies raised against one antigen while the other pair is homologous to those raised against another antigen. Preferably, the antibodies produced and/or administered for use in treating an overdose of an amphetamine, in amphetamine detoxification, and in preventing abuse and/or overdose, are neutralizing antibodies. As used herein, the term antibody refers to entire antibody molecules comprising both heavy and light chains, but also to any fragment of said antibody such as $(Fab)_2$, Fab, Fv fragments and ScFv fragments that retain the desired specificity of binding to amphetamines and amphetamine derivatives. As used herein, the term" neutralizing ", when referring to antibodies, means that such antibodies bind to amphetamine or amphetamine derivatives, mainly amphetamine metabolites, found in body fluids of subjects consuming amphetamines to prevent binding of the amphetamine to cellular receptors. Preferably, the antibodies have a sufficient affinity for amphetamines that they can remove receptor bound ligands, amphetamine or amphetamine derivatives, from their receptor, when the ligands are bound to the receptor.

In one embodiment, new compounds are obtained that can induce specific antibodies directed against amphetamine derivatives, when coupled to an appropriate carrier and administrated to an animal. The present invention preferably relates to (S)-enantiomer compounds and antibodies to these compounds, wherein the (S)-enantiomers have the formula (I)

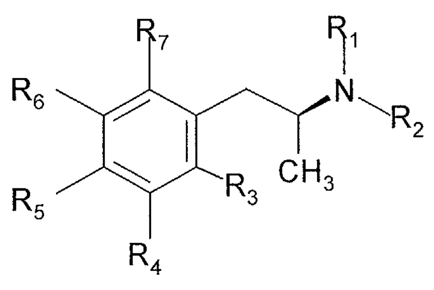

(I)

wherein,

- $R_1$ and $R_3$ are selected from the group consisting of hydrogen, and $C_1$-$C_3$ alkyl,
- $R_2$ is selected from the group consisting of hydrogen, a $C_1$-$C_3$ alkyl and a polymethylene chain: $-(CH_2)_n$-COOH where n is an integer between 1 and 6,
- $R_4$, $R_6$ and $R_7$, identical or different, are selected from the group consisting of hydrogen, halogen, $-OR_9$ and $-SR_9$, wherein $R_9$ is hydrogen or a $C_1$-$C_3$ alkyl,

- $R_5$ is selected from the group consisting of hydrogen, a polymethylene chain: $-(CH_2)_m-R_{10}$ and an oxy-polymethylene chain : $-O-(CH_2)_m-R_{10}$, wherein $-R_{10}$ is selected from the group consisting of carboxyl, thiol, and $-CONHR_{13}SH$, and $-CONHR_{13}SH$, $R_{13}$ being selected from the group consisting of-$CH(COOH)CH_2$- and-$(CH_2)_m$- where m is an integer between 1 and 4 with the proviso that when $R_1$ is hydrogen and $R_2$ is a methyl or when $R_1$ is a methyl and $R_2$ is hydrogen, then $R_5$ is not a polymethylene chain: $-(CH_2)_m-COOH$.

Preferred embodiments of the invention are compounds of formula (II)

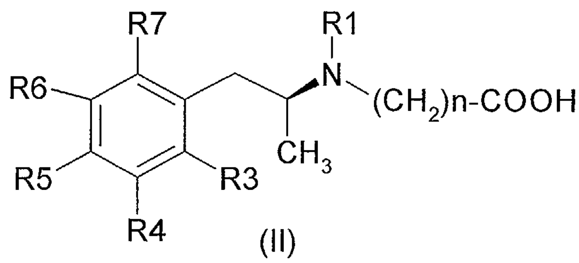

(II)

wherein,

- $R_1$, $R_3$, $R_4$, $R_5$, $R_6$ and $R_7$ have the same meaning as described above, and

wherein n is an integer between 1 and 6.

Yet more preferred embodiments of the invention are compounds of formulas (IIIa)

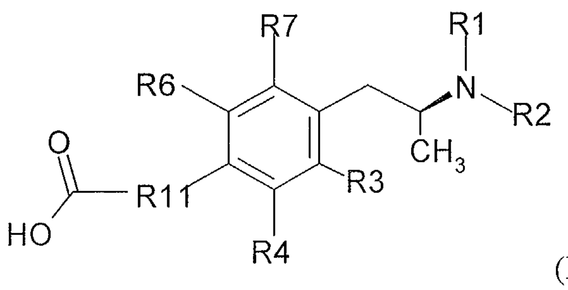

(IIIa)

wherein, $R_1$, $R_2$, $R_3$, $R_4$, $R_5$, $R_6$ and $R_7$ have the same meaning as described above;
- $R_{11}$ is selected from among a polymethylene chain:
$-(CH_2)_m-$ and an oxy-polymethylene chain: $-O(CH_2)_m-$ and m is an integer between 1 and 4, with the proviso that when $R_1$ is a methyl, then $R_{11}$ is not a polymethylene chain: $-(CH_2)_m-$.

Other preferred embodiments of the invention are compounds having formula (IIIb)

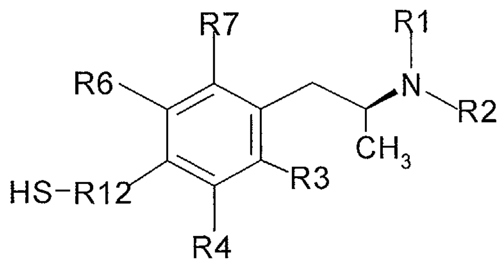

(IIIb)

wherein,- $R_1$, $R_2$, $R_3$, $R_4$, $R_6$, and $R_7$ have the same meaning as described above, and $R_{12}$ is selected from from the group consisting of a polymethylene chain: $-(CH_2)_mCONHR_{13}$ and an oxy-polymethylene chain: $-O(CH_2)_mCONHR_{13}$, $R_{13}$ and m has the same meaning as described above.

Compounds of formulas IIIa and IIIb bear a chain suitable for linkage bonded to the aromatic group of the amphetamine. This linkage is most preferably bonded by means of an oxygen atom so that the antibodies produced against an immunogen bearing such a compound can neutralize amphetamines substituted on the aromatic ring with heteroatoms (e.g., ecstasy, 4-methoxyamphetamine and 4-methylthioamphetamine.)

Particularly preferred embodiments of the invention are compounds of formula (IV), (V), (VI) and (VII). Compound VII also is referred to as MET1.

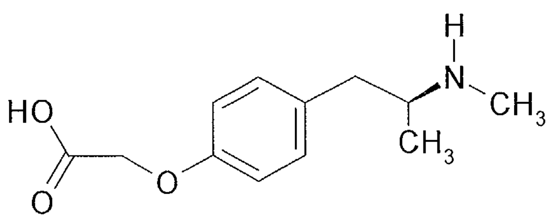

(IV)

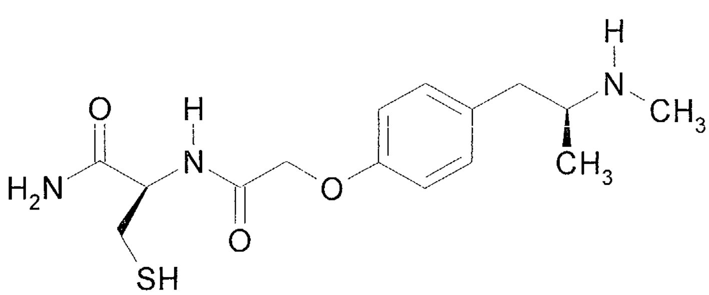

(V)

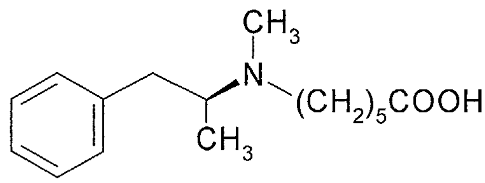

(VI)

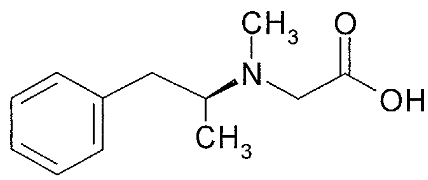

(VII)

Another object of the invention is a method to prepare these new amphetamine derivatives. Synthesis of the compounds has been achieved by introducing spacers at two different positions of the amphetaminic skeleton.

Typically, such a method, as illustrated in Figure 1, comprises the following steps:

(a) Alkylation of a methamphetamine derivative having the formula (IX), wherein $R_1$, $R_3$, $R_4$, $R_5$, $R_6$, and $R_7$ have the same meaning as described above, by addition of $Br(CH_2)_nCOOBn$, where n is an integer between 1 and 6, in an appropriate solvent, to obtain an addition product of formula (X), wherein $R_1$, $R_3$, $R_4$, $R_5$, $R_6$, and $R_7$ have the same meaning as described above and n has the same value as described above.

(b) Hydrogenation of said addition product of formula (X) to obtain a compound of formula (II).

Also the invention relates to a method to prepare compounds having formula (IIIa), as illustrated in Figure 2, comprising the following steps:

(a) condensation of the aldehyde derivative having formula (XI) wherein $R_3$, $R_4$, $R_6$ and $R_7$ have the same means as previously defined with $CH_3CH_2$-$NO_2$ to obtain the nitrostyrene derivative of formula (XII);

(b) reduction of the nitrostyrene derivative (XII) into the amphetamine derivative (XIII);

(c) alkylation of the primary amine of the compound (XIII) leading to the formation of compound (XIV);

(d) protection of the amine group of compound (XIII) or compound (XIV) by acylation with ditertbutyldicarbonate to obtain the protected derivative (XV);

(e) removal of the benzyl group of compound (XV) by hydrogenation using Pd/C as catalyst leading to the formation of compound (XVI);

(f) alkylation of the phenol groups of compound (XVI) by reaction with benzylbromoalkylcarboxylate to obtain the benzylester compound (XVII);

(g) catalytic hydrogenation of compound (XVII) to afford to the carboxylic derivative (XVIII); and

(h) deprotection of the amino group of compound (XVIII) by reaction with TFA, followed by separation of both enantiomers obtained on a chiral HPLC column to obtain a compound of formula (IIIa).

Also the invention relates to a method to prepare compounds having formula (IIIb), as illustrated in Figure 2, comprising the following steps:

(a) Condensation of the aldehyde derivative having formula (XI) wherein $R_3$, $R_4$, $R_6$ and $R_7$ are as previously defined with $CH_3CH_2$-$NO_2$ to obtain the nitrostyrene derivative of formula (XII);

(b) reduction of the nitrostyrene derivative (XII) into the amphetamine derivative (XIII);

(c) alkylation of the primary amine of the compound (XIII) leading to the formation of compound (XIV);
(d) protection of the amine group of compound (XIII) or compound (XIV) by acylation with ditertbutyldicarbonate to obtain the protected derivative (XV);
(e) removal of the benzyl group of compound (XV) by hydrogenation using Pd/C as catalyst leading to the formation of compound (XVI);
(f) alkylation of the phenol groups of compound (XVI) by reaction with benzylbromoalkylcarboxylate to obtain the benzylester compound (XVII);
(g) catalytic hydrogenation of compound (XVII) to produce the carboxylic derivative (XVIII);
(h) acylation of (R)-Cysteine derivative, (R)H-cys(Otrt)-$NH_2$, by (XVIII) using coupling agents (DCC, HOBt) followed by separation of the obtained diastereoisomer mixture by chromatography on a silica gel column to obtain the S-isomer (XIX); and
(i) elimination of the acid-sensitive protecting groups, triphenylmethyl and BOC of compound (XIX), by Trifluoroacetic acid (TFA) leading to the compound (IIIb).

In order to accomplish acylation step (c) for both above methods, two reaction sequences were found to be efficient:

(a) alkylation of the amine (XIII) with trifluoroacetic anhydride followed by alkylation of the obtained trifluoroacetamide with an alkyl halide (e.g.: methyliodide), followed by removal of the trifluoroacetyl group in basic medium or
(b) alkylation of the amine (XIII) with an acid anhydride (e.g.: formylacetyl mixed anhydride. Acetic anhydride) followed by the reduction of the amide by lithium aluminium hydride.

Another alternative method to prepare compounds of formula (IIIa), as illustrated in Figure 3, comprises the following steps:

(a) reduction of the tyrosine ester derivative of formula (XXII) to obtain the alcohol derivative of formula (XXIII),
(b) alkylation of compound (XXIII) to obtain the compound of formula (XXIV),
(c) removal of the benzyl group of compound (XXIV) by hydrogenation using Pd/C as catalyst leading to the formation of the phenol derivative of formula (XXV),
(d) acylation of the phenol group of compound (XXV) by benzylbromoalkylcarboxylate leading to the formation of the ester derivative (XXVI),
(e) catalytic hydrogenation of compound (XXVI) to obtain the compound (IIIa).

Another aspect of the invention relates to immunogens bearing compounds of the invention for eliciting an immune response, when provided to an animal. Immunogens of the invention are obtained by covalent coupling of the compounds of the invention with an appropriate carrier by using coupling agents and reactions well known by those skilled on the art. The carrier used to prepare an immunogen is a molecule containing at least one T cell epitope which is capable of stimulating the T cells of the mammal to be immunized. Preferably, the carrier, like the hapten, should be sufficiently foreign to elicit a strong immune response to the vaccine and to avoid the phenomenon of carrier-induced epitope suppression. It therefore is preferable to use as a carrier a molecule to which the intended recipient has not been exposed. Suitable carrier molecules include bacterial toxins or products, for example, cholera toxin B-(CTB), diphtheria toxin, tetanus toxoid, and pertussis toxin and filamentous hemagglutinin, shiga toxin, and Pseudomonas exotoxin; lectins such as the ricin-B subunit, abrin and sweet pea lectin; viral proteins such as retrovirus nucleoprotein (retro NP), rabies ribonucleoprotein (rabies RNP), plant viruses (e.g. TMV, cow pea and cauliflower mosaic viruses), vesicular stomatitis virus-nucleocapsid protein (VSV-N), poxvirus vectors and Semliki forest virus vectors; and malarial protein antigen and peptidic fragments of any of the above. In countries where standard childhood immunizations include diphtheria and tetanus, proteins such as tetanus toxoid and diphtheria toxoid, if unmodified, may be less desirable as appropriate carriers for immunogens for use in humans. Likewise, bovine serum albumin would be less desirable as a carrier for immunization of humans in regions where beef is included in the diet of most humans. Still further, it is highly advantageous if the carrier has inherent immunogenicity/adjuvanticity and/or is capable of eliciting both a systemic response and response at the site of amphetamine exposure.
Amphetamines generally are taken orally so a preferred carrier elicits not only a systemic response but also a pre-existing mucosal antibody response. In such a mucosal response the reaction of antibodies with amphetamine happens rapidly enough to counteract the drug before it begins circulating in the blood stream. One such ideal carrier is cholera toxin B (CTB), a highly immunogenic protein subunit capable of stimulating strong systemic and mucosal antibody responses. The B subunit of cholera toxin also can serve both as a targetting molecule for the hapten for M cells in the intestinal lining. CTB has already been shown to be safe for human use in clinical trials for cholera vaccines (Holmgren et al., (1994) Am. J. Trop. Med. Hyg. 50: 42-54; Jertborn et al. (1994) Vaccine 12:1078-1082; "The Jordan

Report, Accelerated Development of Vaccines" 1993., NIAID, 1993). Other useful carriers that can enhance a mucosal response, include the LTB family of bacterial toxins, retrovirus nucleoprotein (retro NP), rabies ribonucleoprotein (rabies RNP), vesicular stomatitis virus-nucleocapsid protein (VSV-N), recombinant pox virus subunits, and multiantigenic peptides (MAP).

Coupling reactions between carrier and compounds of the invention can be performed using well known reactions between primary amines, carboxylic and thiol groups such as mixed anhydride method, carbodiimide, etc. Generally, the coupling step involves the dehydrative coupling of a free carboxyl of one reactant with the free amino group of the other reactant in the presence of a coupling agent to form a linking amide bond. Coupling can be obtained commercially, for example, from Pierce Chemical Company USA. Examples of suitable coupling agents include carbodiimide or N, N'-dicyclohexylcarbodiimide, 1-hydroxybenzotriazole in the presence of N,N'-dicyclohexylcarbodiimide or N-ethyl-N'-[(3-dimethylamino)propyl]carbodiimide. A practical coupling agent is the commercially available (benzotriazol-1-yloxy)tris-(dimethylamino)phosphonium hexafluorophosphate, either by itself or in the presence of 1-hydroxybenzotriazole. Other coupling agents that find use with the subject invention and are commercially available include 2-(1H-benzotriazol-1-yl)-N, N, N', N'-tetramethyluronium tetrafluoroborate and O-(7-azabenzotriazol-1-yl)-N,N,N',N'-tetramethyluronium hexafluorophosphate. Typically, coupling reactions are conducted in an inert solvent such as dichloromethane, acetonitrile or dimethylformamide. An excess of a tertiary amino, e.g. diisopropylethylamino, N-methylmorpholine or N-methylpyrrolidine, is added to maintain the reaction mixture at a pH of about 8. The reaction temperature usually ranges between 0° and 50° C. The reaction time usually ranges between 15 min and 24 h. These coupling reactions can be performed in either a liquid or a solid phase.

The invention also concerns immunogens having the formula I1 (see below) coupled to a carrier. These immunogens may be prepared, for example, by linking a compound of formula I1, wherein $R_2$ of formula I1 is $-(CH_2)_n-COOH$ to a carrier with the carbodiimide method. 1-Ethyl-3-(3-dimethylaminopropyl) carbodiimide), reacts with a carboxylic group, allowing it to be coupled to the amino group in the reaction mixture. The carbodiimide method thus activates the side chain carboxylic group so that reacts with primary amines on the carrier. The activated compound is mixed with a carrier protein such as, for example, cholera toxin B subunit, to produce the final conjugate. Descriptions of the carbodiimide method and other coupling agents and methods may be found in, for example, M. Bodanszky, "Peptide Chemistry", 2nd ed., Springer-Verlag, Berlin, Germany, (1993). Examples of carrier bound immunogens are shown below. Immunogens of formula I1:

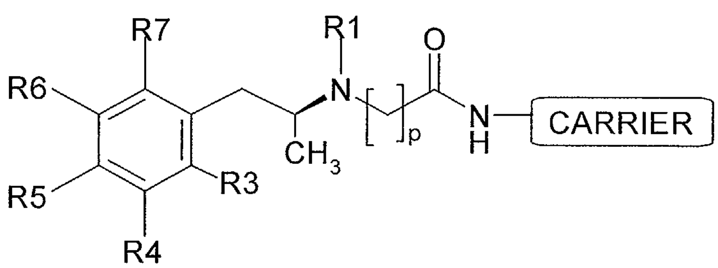

I1

wherein $R_1$, $R_3$, $R_4$, $R_5$, $R_6$ and $R_7$ are as described previously, n is an integer between 1 and 6; and p is an integer between 1 and 6; immunogens of formula 12:

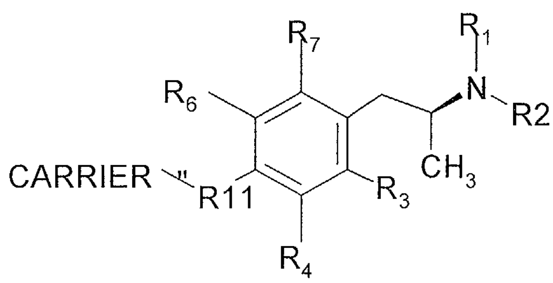

I2

wherein $R_1$, $R_2$, $R_3$, $R_4$, $R_6$, $R_7$ and $R_{11}$ are as described previously and; immunogens of formula 13:

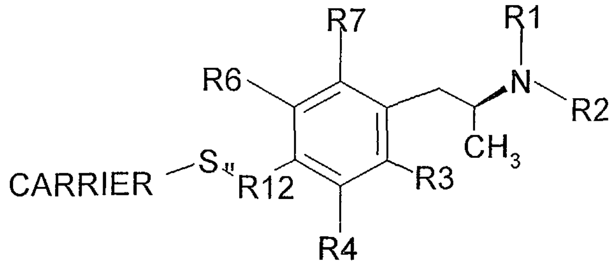

I3

wherein $R_1$, $R_2$, $R_3$, $R_4$, $R_6$, $R_7$ and $R_{12}$ are as described above.

Another object of the present invention is the use of immunogens such as I1, I2 and I3 for the production of monoclonal and polyclonal antibodies that specifically recognize amphetamine derivatives. Another aspect of the present invention relates to antibodies that can neutralize amphetamine or amphetamine related drugs after their abuse by users of these drugs. Thus, the present invention offers a new therapeutic approach for treating prophylactically amphetamine addiction in particular, in the form of an amphetamine-related immunogen that can be used to elicit an immune response leading to production of anti-amphetamine antibodies by the abuser, which in case of drug intake can neutralize the amphetamines ingested and thereby reduce or suppress the pharmacologic effects of the abused drug.

A main object of the invention then is to provide new pharmaceutical compositions useful for treating amphetamine abuse. The invention relates to pharmaceutical compositions comprising at least one amphetamine-related immunogen of the invention to prepare a vaccine for the treatment and/or prevention of amphetamine abuse pathologies. The administration to a patient, suffering from amphetamine abuse, of a pharmaceutical composition, comprising the amphetamine-related immunogens of the invention leads to the development of a passive immunization with the induction of human specific antibodies that neutralize the related drugs. Therapeutic compositions of the invention include compositions containing an amphetamine related immunogen in a pharmaceutically acceptable formulation such as a saline solution at concentrations between about 1 and 100 mg/mL, preferably between about 10 and 60 mg/L.

The invention relates also to antibodies that neutralize amphetamines of abuse such as ecstasy and methamphetamine and more particularly to antibodies that neutralize more than one amphetamine or amphetamine derivative simultaneously.

Preferably the invention relates to antibodies having an affinity for at least one of the immunogens of the invention, most preferably for at lest two immunogens.

Preferably the antibodies have an affnity constant (Ka) for amphetamine or its structural analogs of at least $10^6M^{-1}$, preferably of at least $10^9M^{-1}$ and most preferably of about $10^{10}M^{-1}$ when measured against compounds of the invention.

The present invention refers also to hybridomas producing and preferably secreting immunoglobulins, preferably murine mainly IgG class immunoglobulins, directed against methamphetamine and its structural analogs. Preferred antibodies are murine monoclonal antibodies obtained by fusion between mouse splenocytes, from mice previously immunized with the above described immunogens, and murine myeloma cells, for example SP2O/Ag-14 cells. After fusion, murine hybridomas are obtained and specifically selected based upon their capacity to produce and secrete murine immunoglobulins, mainly IgG class immunoglobulins, directed against methamphetamine and its structural analogs. Preferred antibodies also are monoclonal antibodies that can neutralize at least two different amphetamine-derivatives, preferably methamphetamine and ecstasy simultaneously. Particular preferred monoclonal antibodies of the invention are DASM243-6H5D1C4 and DASM243-3A10A6A2. Preferred murine hybridomas are murine clones producing and secreting monoclonal antibodies DASM243-6H5D1C4 and DASM243-3A10A6A2. The most preferred hybridoma is: DASM243-645D1C4 having accession number CNCM 1-2750. Hybridoma technology to create "monoclonal" antibodies is well known to those of skill in the art (Kohler, et al., Eur. J. Immunol., 6: 511 (1976)). In this process, splenocytes or lymphocytes from an animal which has been injected with antigen are fused with a tumor cell line, thus producing hybrid cells or "hybridomas" which are both immortal and capable of producing the genetically coded antibody of the B cell. The hybrids thus formed are segregated into single genetic strains by selection, dilution, and regrowth, and each strain thus represents a single genetic line. They therefore produce immunoreactive homogeneous antibodies against a desired antigen. Hybridoma technology generally uses fusion of murine lines, but human-human hybridomas (Olsson, L. et al., Proc. Natl. Acad. Sci. (USA), 77: 5429 (1980)); human-murine hybridomas (Schlom, J., et al. (ibid) 77: 6841 (1980)) and several other xenogenic hybrid combinations also been reported.

Antibodies also can be prepared by recombinant means. Messenger RNA coding for a heavy or a light chain

is isolated from a suitable source, such as mature B cells or a hybridoma culture making antibodies of the desired specificity using standard techniques of RNA isolation, and the use of oligo-dT cellulose chromatography to segregate the poly-A mRNA. The poly-A mRNA may be fractionated to obtain sequences of sufficient size to code for the amino acid sequences in the light or heavy chain of the desired antibody. A cDNA library is then prepared from the mixture of mRNA using a suitable primer, preferably a nucleic acid sequence which is characteristic of the desired cDNA. Such a primer may be hypothesized and synthesized based on the amino acid sequence of the antibody if the sequence is known. In the alternative, cDNA from unfractionated poly-A mRNA from a cell line producing the desired antibody or poly-dT also can be used. Cloning vectors containing the resulting cDNA are prepared and used to transform a suitable host cell strain, typically E. coli. Successful transformants are identified by means of, for example, tetracycline resistance or other phenotypic characteristic residing on the cloning vector plasmid. The transformant cultures are then probed with suitable nucleotide sequences containing bases known to be complementary to desired sequences in the cDNA. Plasmids from clones which successfully hybridize are isolated and sequenced by means known in the art to verify that the desired portions of the gene are present. The desired gene fragments are excised and tailored to assure appropriate reading frame with the control segments when inserted into suitable expression vectors. The tailored gene sequence is then positioned in a vector which contains a promoter in reading frame with the gene and compatible with the proposed host cell. A number of plasmids which already contain the appropriate promoters, control sequences, ribosome binding sites, and transcription termination sites, as well as convenient markers are known to those of skill in the art.

The genes also may be tailored to produce modified antibodies. For example, a mammalian heavy chain may not be derived entirely from a single source or single species, but portions of a sequence can be recovered from differing pools of mRNA, such as murine-murine hybridomas, human-murine hybridomas, or B cells differentiated in response to a series of antigen challenges. The desired portions of the sequences in each case can be recovered using the probe and analysis techniques described above, and recombined in an expression vector. Such chimeric chains can be constructed of any desired length; hence, for example, a complete heavy chain can be constructed, or only a sequence for the Fab region thereof. Composite antibodies also can be prepared. For example, in order to make an anti-methamphetamine light chain/anti-ecstasy heavy chain composite antibody, a suitable source for the mRNA used as a template for the light chain clone comprises an anti-methamphetamine producing cell line. The mRNA corresponding to the heavy chain is derived from B cells raised in response to ecstasy or from an anti-ecstasy producing hybridoma.

For construction of chimeric antibodies in which for example, the variable sequences are separately derived from the constant sequences, desired portions of the genes encoding for parts of the heavy and light chains from suitable, differing, sources are recovered and then ligated to reconstruct the gene coding for each chain. For example, portions of the heavy chain gene and of the light chain gene which encode the variable sequences of antibodies produced by a murine hybridoma culture are cloned and gene fragments encoding the constant regions of the heavy and light chains for human antibodies are cloned from, for example, human myeloma cells. The variable portions of the mouse gene are then ligated to the constant regions of the human gene for each of the two chains. Rather than splicing portions of the chain(s), suitable amino acid alterations, deletions or additions are made using available techniques such as mutagenesis, to provide for desiredcharacteristics.

The gene coding for the light chain and that coding for the heavy chain can be inserted into separate expression plasmids, or together in the same plasmid, so long as each is under suitable promoter and translation control, and used to transform suitable cells which are grown under conditions appropriate to the production of the desired protein. Such conditions are primarily mandated by the type of promoter and control systems used in the expression vector, rather than by the nature of the desired protein. The protein thus produced is then recovered from the cell culture by methods known in the art, the choice of which is necessarily dependent on the form in which the protein is expressed. When heavy and light chains are coexpressed in the same host, the isolation procedure is designed so as to recover reconstituted antibody. This can be accomplished using methods known to those of skill in the art.

Specific antibody fragments of the invention such as $(Fab)_2$, Fab, and Fv fragments, wherein the specificity against amphetamine and its structural analogs is conserved, can be obtained by chemical cleavage of complete antibodies according to well-known methods (*see* for example Weir (1986). Handbook of Experimental Immunology. 4th Edition. Blackwell, Oxford. Vol. 1. Immunochemistry). $(Fab)_2$, Fab, Fv and ScFv fragments also can be obtained by recombinant technology by cloning genes coding for variable regions of heavy and/or light antibody chains or portions of them bearing the sequences coding for antibody regions specifically recognizing amphetamines or their structural analogs alone or in a recombined form to obtain specific recombinant Fab or ScFv fragments.

Pharmaceutical compositions of the invention are suitable for use in a variety of delivery systems for administration to humans, including administration parenterally, e.g., intravenously, subcutaneously, intradermally, intraperitoneally, or intramuscularly. The antigen formulations can also be delivered using implanted mini-pumps, which are well known to those skilled in the art. The compositions include formulations comprising one or more purified amphetamine-hapten conjugates and/or amphetamine derivative-hapten conjugates as well as antibodies specific for one or more amphetamine and/or amphetamine derivative. The antibodies can be purified from sera from any animal in which

antibodies to amphetamine and amphetamine derivatives can be raised. Preferably the antibodies are humanized. Humanized antibodies may be produced in transgenic animals that produce human antibodies. Humanized antibodies may also be produced by biochemical modification of nonhuman antibodies, for example, murine monoclonal antibodies, which may include fusing the antigen-binding or Fab portion of the murine monoclonal antibody with a non-binding or Fc region of a human antibody. Humanized antibodies generated by these and other methods retain desired antigen binding specificity, generally without causing an undesirable immune response to the antibody itself.

Examples of pharmaceutically acceptable carriers and formulations for use with the compositions of the present invention are found in Remington's *Pharmaceutical Sciences*, Mack Publishing Company, Philadelphia, PA, 17th ed. (1985), which is incorporated herein by reference. For methods for drug delivery, *see* Langer, (1990) *Science* 249:1527-1533, which is incorporated herein by reference. For vaccine use and production, see Plotkin, et al. (eds.) (1999) *Vaccines*, 3rd edition. W.B. Saunders, Philadelphia, and Zegers, et al. (eds.) (1995) Immunological Recognition of Peptides in Medicine and Biology, CRC Press, Boca Raton, Florida, which reference is incorporated herein by reference.. For mucosal vaccine delivery, see Ryan *et. al*., (2001) *Trends Biotechnol* 19: 293-304, and Ogra, *et al*., (2001) *Clin Microbiol Rev* 14:430-445, which are incorporated herein by reference. For examples of adjuvants, see Gregoriadis, G., ed., (1990) Immunological Adjuvants and Vaccines (NATO Asi Series A, Life Sciences, Vol 179), which is incorporated herein by reference.

In preparing pharmaceutical compositions of the present invention, it may be desirable to modify the compositions of the present invention to alter their , immunogenicity and biodistribution. For a general discussion of pharmacokinetics, see Remington's *Pharmaceutical Sciences, supra*, Chapters 37-39. A number of methods for altering pharmacokinetics, immunogenicity and biodistribution are known to one of ordinary skill in the art (*See*, e.g., Langer, *supra*, Gregoriadis, (1990), *supra*). Examples of such methods include protection of the agents in vesicles composed of substances such as proteins, lipids (for example, liposomes), carbohydrates, or synthetic polymers. For example, the vaccines of the present invention can be incorporated into liposomes in order to enhance their immunogenicity and biodistribution characteristics. Liposomes that microencapsulate vaccine antigens, and are then polymer-coated, are useful for controlling the release rate, and hence the efficacy, of parenterally and orally administered vaccines. A variety of methods are available for preparing liposomes, as described in, e.g., Szoka *et al*, (1980) *Ann. Rev. Biophys. Bioeng*. 9:467, U.S. Pat. Nos. 4,235,871, 4,501,728 and 4,837,028, all of which are incorporated herein by reference. For a brief review of the use of liposomes as antigen-entrapping and delivering adjuvants or immunomodulators, see Gregoriadis (1999) *Methods* 19:156-162, and Rogers, et. al. (1998) *Crit Rev Ther Drug Carrier Syst* 15: 421-480, which are incorporated herein by reference. Polymeric lamellar substrate particles produced by precipitation of poly(D,L-lactide) form a polymeric system for the adsorption of antigens. This procedure avoids pH changes, exposure to organic solvents and hence allows the integrity of the antigen to be retained. For polymeric lamellar substrate particles for intranasal vaccination, *see* Jabbal-Gill *et al*. (2001) *Adv Drug Deliv Rev* 51: 97-111, which is incorporated herein by reference.

To prepare formulations for injection, a solution of the composition is dissolved or suspended in an acceptable carrier, preferably an aqueous carrier. A variety of pharmaceutically acceptable aqueous carriers can be used, e.g., water, buffered water, 0.4% saline, 0.85% saline solution, 0.3% glycine, hyaluronic acid and the like. The conjugate formulations may also comprise an adjuvant to stimulate an active immune response to the antigen. Examples of adjuvants are well known in the art and include, for example, aluminum hydroxide (Spectrum Chem. Mtg. Corp., New Brunswick, N.J.) or aluminum phosphate (Spectrum),calcium phosphate, saponins, monophosphoryl lipid A, Freunds adjuvant, liposomes, polymer-coated liposomes, polymeric lamellar substrate particles, and cytokines such as interleukin-2.

The compositions can contain as pharmaceutically acceptable carriers, substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, and the like, as well as preservatives including, for example, thimerisol, and protein carriers including, for example, human serum albumin or animal sera. The compositions can be sterilized by conventional, well-known sterilization techniques, including sterile filtration. The resulting aqueous solutions or suspensions can be packaged for use as is, or lyophilized, the lyophilized preparation being combined with a sterile solution prior to administration. For solid compositions, conventional nontoxic pharmaceutically acceptable carriers can be used which include, for example, pharmaceutical grades of mannitol, lactose, starch, magnesium stearate, sodium saccharin, talcum, cellulose, glucose, sucrose, magnesium carbonate, and the like.

Oral vaccines can be prepared in several ways, for example, as aqueous solutions, suspensions, tablets, pills, capsules, or powders, as well as sustained release formulations such ascontrolled-release microcapsule pharmaceutical formulations for sustainable, programmable release of an antigen, including biodegradable poly(lactide/glycolide) or poly(lactide. generally contain from about 10% to about 95% of the active ingredient, preferably about 25% to about 70%.). Oral vehicles may include excipients such as, for example, mannitol, lactose, starch, magnesium carbonate, magnesium stearate, and sodium saccharin cellulose. Sustained or controlled release formulations can be

prepared by incorporating the composition into liposomes, nonresorbable impermeable polymers such as ethylenevinyl acetate copolymers, swellable polymers such as hydrogels, or resorbable polymers such as collagen and certain polyacids or polyesters.

The oral vaccines also can be prepared as foods, including for example sugar cubes, and as edible portions of transgenic plants that have been genetically modified to express an antigen in an edible portion of the plant. The edible portions can be unprocessed such as fruit or vegetables, preferably raw or as products prepared from the edible portions. Charles Arntzen and others found that transgenic plants expressing desirable immunogens may, when ingested, produce an immune response to the immunogens. *See* for example Lam, et al., (1996) U.S.P.N. 5,484,719, Lam, et al., (1997) U.S.P.N. 5,612,487, Arntzen, et al., (1998) U.S.P.N. 5,792,935, Arntzen, et al., (1999) U.S.P.N. 5,914,123, Lam, (2000) U.S.P.N. 6,034,298, Arntzen, et al., (2000) U.S.P.N. 6,136,320, Arntzen, et al., (2001) U.S.P.N. 6,194,560, and Arntzen, et al, (1994) *Vaccines* 339-344). To date, vaccines have been produced in bananas, potatoes, tomatoes, lettuce, rice, wheat, soybeans and corn (Langridge (2000) Sci Am 283: 66-71).

Intranasal formulations generally comprise vehicles that do not cause significant irritation to the nasal mucosa or disturb ciliary function. Diluents may include water and saline solutions. Nasal formulations may also contain preservatives including chlorobutanol and benzalkonium chloride. A surfactant or other penetration enhancer may be present to enhance absorption of the subject proteins by the nasal mucosa. See for example USPN 6,136,606; USPN 6,077,516; USPN 6,077,514; 6,048,536; 5,932,222; and USPN 5,756,104.

For aerosol administration, the pharmaceutical compositions are preferably supplied in finely divided form along with a surfactant and propellant in pharmaceutically acceptable carriers. The surfactant should be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides, can be employed. A carrier also can be included, as desired, as with, for example, lecithin for intranasal delivery. The formulations also can be administered in the form of suppositories. Suppositories generally include binders and carriers, including, for example, polyalkaline glycols and triglycerides. Suppositories may be formed from mixtures containing an antigen in the range of about 0.5% (w/w) to about 10% (w/w), preferably from about 1% (w/w) to about 2% (w/w).

The present invention also relates to a method of treating a patient who is abusing amphetamines by administering an appropriate dose of an amphetamine-or amphtamine-related immunogen The amount of antigen in each vaccine dose is selected as an amount which induces an immunoprotective response without significant, adverse side effects in typical vaccinees. Such amount will vary depending on which specific immunogens are employed. Generally it is expected that each dose will comprise about 1-1000 μg of total immunogen, preferably about 2-100 μg, more preferably about 1-40 μg, and most preferably about 1-5 μg. An optimal amount for a particular vaccine can be ascertained by standard studies involving observation of antibody titers and other responses in subjects. A primary vaccination course can include 2 or 3 doses of a vaccine, given one to two months apart, however, due to genetic and other factors, an antibody response to any vaccine preparation is likely to vary between preparations and individuals. Response to vaccination with the anti-amphetamine or anti-amphetamine derivative conjugates is monitored to determine both short and long-term vaccine efficacy, the former can be used for evaluating conjugate preparations and the latter for evaluating individuals as to their need for "booster" vaccinations. To determine antibody titer following vaccination with various proteins conjugated with amphetamine derivatives, blood specimens from vaccinated adults are analyzed using standard techniques known to those skilled in the art such as by ELISA. Preferably, the blood specimens include samples from each patient before (i.e., a negative control) and about four weeks after vaccination and subsequently at intervals of about 2 to 6 months. As needed, a booster vaccination can be administered.

The compositions also can be used to provide passive immune protection against amphetamine toxicity by administering purified antibodies obtainable from sera from animals vaccinated with amphetamine conjugate preparations. The efficacy of protection can be determined by an increase in the amount of the abused amphetamine or amphetamine derivative that is required to produce a physical symptom associated with abuse of amphetamine or a derivative thereof, such as increased blood pressure, increased heart rate, or pupil dilation. A purified antibody formulation also can be used to treat a patient exhibiting symptoms associated with amphetamine abuse or amphetamine derivative abuse, as well as patients exhibiting symptoms associated with overdose. The dose of the antibody formulation provided should be an amount that is capable of binding a sufficient amount of free amphetamine and/or amphetamine derivatives to alleviate the patient's symptoms, generally an amount that binds substantially all free amphetamine in the patient's blood. The antibodies can be provided by direct administration to the patient or by passing the patients blood through an extracorporeal device that comprises the antibodies which optionally also can include activated charcoal. Efficacy of treatment can be monitored by a conteraction of symptoms associated with abuse and/or overdose.

The antibodies used in the formulations for both chronic and acute treatment regimens preferably bind to at least 2 to 4, more preferably at least 2-6 and most preferably to 2-10 amphetamines that are typically abused and thereby provide protection or relief of symptoms against a wide range of amphetamines and their derivatives. Preferably

at least two of the analogs to which the antibodies bind are ecstasy, 4-methylthio-amphetamine, metamphetamine, or N-ethylamphetamine. Also preferably, the antibodies have high affinity for amphetamine compounds, preferably about $10^6M^{-1}$ and above and more preferably about $10^8M^{-1}$ and above and have minimal affinity, preferably less than about $10^5M^{-1}$ and more preferably less than about $10^3M^{-1}$ for biologically inactive amphetamine metabolites. Conveniently, the formulations can be provided in single dose kits in sterile vials so that the physician may employ the vials directly, where the vials will have the desired amount and concentration of formulation. When the vials contain the formulation for direct use, usually there will be no need for other reagents for use with the method. The subject compositions can be contained in packaging material, which comprises a label indicating that the subject compositions can be used to treat amphetamine and amphetamine analog abuse and/or overdose in humans.
The following examples are provided by way of illustration, not limitation.

EXAMPLES

Example 1

Synthesis of amphetamine derivatives

The synthesis of compounds of the invention of formula (II) are detailed in examples hereafter described and illustrated in Figure 1. The numbers refer to the steps shown in the figures.

1.1. Chemistry

1.1.1. Preparation of haptens type II.

1.1.1.1. Alkylation of methamphetamine.

Method 1.

DMAP (2.44 g ) was added to a solution of d-methamphetamine 1 (Compound (IX) wherein $R_1$= $-CH_3$), $R_3=R_4=R_5=R_6=R_7=H$) purchased from Sigma (3 g) in THF. $BrCH_2COOBn$ (3.2 mL) in THF then was added drop by drop. The reaction mixture was stirred for 2 hours.
The purified product 2a (Compound (X), wherein $R_1$= $-CH_3$, n=1 and $R_3=R_4=R_5=R_6=R_7=H$) was obtained by column chromatography using toluene/ethyl acetate as eluents.
The hydrogenation of the product was carried out by using $H_2$/Pd in a solution of ethanol. The solid obtained was recrystallized from isopropanol, white crystals or plates of 3a (Compound (II), wherein $R_1$= $-CH_3$, n=1 and $R_3=R_4=R_5=R_6=R_7=H$) were obtained.

1.1.1.2. Alkylation of methamphetamine.

Method 2

A solution of NaI (0.262 g) and $Br(CH_2)_5COOBn$ (0.5 g) in acetonitrile 20 mL was heated at 60°C for 30 minutes. To this solution was added $K_2CO_3$ (0.258 g) and d-methamphetamine The mixture was then refluxed at 60°C for 3h.
After completion of the reaction, the reaction mixture was extracted with ethyl acetate to obtain product 2b (Compound (X) wherein $R_1$= $CH_3$, n=5 and $R_3=R_4=R_5=R_6=R_7=H$). The reduction of product D was carried out, to obtain Product 3b (Compound (II), wherein $R_1$= $-CH_3$, n=5 and $R_3=R_4=R_5=R_6=R_7=H$)(2.3 g) was isolated as an oil.
The hydrochloride was formed by anhydrous gaseous hydrochloric acid in an ether solution.

1.1.2. Preparations of haptens type III

The synthesis of compounds of the invention of formula (IIIb) are detailed in examples hereafter described and illustrated in Figures 2 and 3.

1.1.2.1 Synthesis of 1-benzyloxy-4-(2-nitroprop-1-enyl)benzene 5 (Compound) (XII) wherein Bn=$-CH_2-C_6H_5$, and $R_3=R_4=R_6=R_7=H$).

A solution of 4-benzyloxybenzaldehyde 4 (Compound) (XI), wherein Bn=$-CH_2-C_6H_5$, and $R_3=R_4=R_6=R_7=H$)

(25 g) and ethylenediamine (0.5 mL) in nitroethane (125 mL) was heated to reflux for 5-6 h. On cooling, yellow crystals of compound 5 deposited and were filtered, washed with a 10 mL methanol, and dried to give (24.76 g) yield 77 %, m. p. 143-145 °C $^1$H NMR ($CDCl_3$) ppm: 2.41 □(s □□3H, $-CH=CCH_3NO_2$), 5.05 (s, 2H, $C_6H_5CH_2O$-), 7.4 (d, 2H *J*=8Hz), 6.95 (d, 2H, *J*=8Hz) 8.00 (s, 1H, $-CH=CCH_3NO_2$). Anal. ($C_{16}H_{15}NO_3$) C, H, O, N.

1.1.2.2 Synthesis of 2-(4-benzyloxy-phenyl)-1-methyl-ethylamine 6a (Compound) (XIII) wherein Bn=$-CH_2-C_6H_5$, $R_3=R_4=R_6=R_7$=H).

Lithium aluminum hydride (22.71 g) was placed in a three necked round bottom flask. Then anhydrous THF was added drop by drop and the reaction was carried out in an ice bath. The 1-benzyloxy-4-(2-nitroprop-1-enyl)benzene (24.76 g) in THF was added drop by drop; when the addition was finished, the reaction mixture was stirred for one hour. It was then refluxed for 6 hours at 60-70°C.

After completion of the reaction, the reaction mixture was left to return to rt before carrying out the hydrolysis, to obtain compound 6a (21.13 g) yield 85.5%. $^1$H NMR ($CDCl_3$) δ ppm: 1.05 (d, *J*=6.71 Hz, 3H, $-CH-CH_3$-) 1.35 (s, 2H, $NH_2$) 2.1(s, 2H, $-CH_2CH$-) 2.4 (dd, 1H, $CH-CH_3$-) 2.51 (dd, 1H, $-CH_2-CH$-) 4.95 ($C_6H_5CH_2O$-) 6.95 (d, 2H, *J*=8.5Hz) 7.4 (m, H aromatic) Anal. ($C_{16}H_{19}NO$) C, H, O, N. Product 6a was converted into 6b (Compound (XIV) wherein Bn=$-CH_2-C_6H_5$, R1= $-CH_3$ and $R_3=R_4=R_6=R_7$=H) in 3 steps: Acylation of 6a with trifluoroacetic anhydride was followed by alkylation with methyliodide. Finally, the trifluoroacetyl group was removed in basic medium.

1.1.2.3. Synthesis of compound 7

Example of 7a (Compound (XV) wherein Bn=$-CH_2-C_6H_5$, and $R_1=R_3=R_4=R_6=R_7$=H).

Compound 6a (21.13 g) in $CH_2Cl_2$ and THF and $Et_3N$ (12.2 mL) were placed in a round bottom flask and the reaction carried out at 0°C. The $(BOC)_2O$ (31.3 g, 1.5eq.) in THF was then added drop by drop. After 5 h at room temperature, the solvent was evaporated and extracted with ethyl acetate and HCl (2N). The extract was dried with ($Na_2SO_4$), and evaporated, and the residue was purified by chromatography (cyclohexane, then ethyl acetate) to give compound 9 (17.5g), yield 58 % $^1$H NMR ($CDCl_3$) δ ppm 1.0 (d, 3H, $-CH-CH_3$-) 1.35 (s, 3H, $-OCH_3$-) 3.25 (-NH-, 1H) 2.5 ($-CH_2CHCH_3$-) 4.95 ($C_6H_5CH_2O$-) 7.4 (m, H aromatic) Anal. ($C_{21}H_{27}NO_3$) C, H, O, N.

1.1.2.4 Preparation of phenols 8 by removal of benzyl group by catalytic hydrogenation of products 7.

Example of 8a (Compound (XVI, wherein Bn=$-CH_2-C_6H_5$, and $R_1=R_3=R_4=R_6=R_7$=H).

Compound 7a (17.5 g) was dissolved in ethanol and reduced under pressure using Pd-C 10% catalyst by passing hydrogen gas (6 bars, 23°C) After the completion of the reaction, the solution was filtered over a micropore filter. The product was isolated by evaporation of the solution to give compound 8a (11.2 g) yield 94.8 %, $^1$H NMR ($CDCl_3$) δ ppm: 1.05 (d, *J*=6.71 Hz, 3H, $-CH-CH_3$-) 1.35 (s, 9H, $-OC(CH_3)_3$) 2.5 (dd, 1H, *J*=7.32 Hz) 3.27 (NH, 1H) 6.69 (d, 2H, *J*=8.54 Hz) 6.95 (d, 2H, *J*=8.54 Hz) Anal. ($C_{12}H_{21}NO_3$) C, H, O, N

1.1.2.5 Synthesis of compounds 9 by alkylation of 8.

Example of 9a (Compound) (XVII) wherein Bn=$-CH_2-C_6H_5$, and $R_1=R_3=R_4=R_6=R_7$=H).

To suspension of 8a (9.48 g) and potassium carbonate (5.2 g) and methyltrioctylammonium chloride (0.5g) Benzyl bromacetate was added and the reaction mixture refluxed for 6 hours at 60°C.

After completion of the reaction, the solvent was evaporated and the residue extracted with ethyl acetate and water (3x20 mL). The resulting extract was dried with $Na_2SO_4$, and evaporated. The residue was purified by chromatography (toluene, then ethyl acetate) to give compound 9a (6.6 g), yield 54.3%, $^1$H NMR ($CDCl_3$) δ ppm: 1.05(d, *J*=6.71 Hz, 3H, $-CH-CH_3$-) and 1.45 (s, 9H), $-OC(CH_3)_3$) 2.1 (m, 1H, $-CH_2CHNH$-) 5.15 (s, 2H, $C_6H_5CH_2O$-) 6.71 (d, 2H, *J*=8.54 Hz)

1.1.2.6 Removal of benzyl group by catalytic hydrogenation

Example of 10a (Compound (XVIII) wherein Bn=$-CH_2-C_6H_5$, and $R_1=R_3=R_4=R_6=R_7$=H).

The compound 9a (7.6 g) was dissolved in ethanol and reduced under pressure using Pd-C 10% catalyst by passing hydrogen gas (6 bars, 23°C) After the completion of the reaction, the solution filtered over micropore filter. The product was isolated by evaporation of the solution to give compound 10a (3.6 g) yield 71.5%, $^1$H NMR ($CDCl_3$) □□□□□□ (d, *J*=6.71 Hz, 3H, $-CH-CH_3$-) 1.35 (s, 9H, $-OC(CH_3)_3$) 2.5 (dd, 1H, $-CH_2CHNH$-) 3.35 (1H, -NH-) 4.51 (s, 2H, $HOOCCH_2O$-) 6.75 (d, 2H, *J*=8.54 Hz) 7 (d, 2H, *J*=8.54 Hz)

1.1.2.7 Synthesis of compound 11 by acylation of a deprotected cysteine.

Example of 12a (Compound (IIIb) wherein $R_3=R_4=R_6=R_7=H$ and $R_{12}=-O-(CH_2)_m-CONHCONH_2$).

In a round bottom flask, compound 10a (1.6g) was dissolved in ethyl acetate; after 5 min of stirring, HOBt (0.0263 g, 1.3 equivalent) was added at 0°C then DCC (0.4 g, 1.3 equivalent) was added. Dicyclohexylurea which precipitated was removed by filtration.

H-Cys(Trt)-$NH_2$, (0.5 g ) in ethyl acetate was placed in a round bottom flask, the filtrate was added and then triethylamine (0.25 mL) was added.

The reaction mixture was stirred for one day and monitored by T.L.C Toluene/Ethyl acetate 7/3. The solvent was evaporated from the mixture and the residue in ethyl acetate was washed with an alkaline solution.

The organic phase was collected, dried with sodium sulphate and evaporated.

Pure compound 11a (Compound (XIX) wherein $R_1=R_3=R_4=R_6=R_7=H$) (0.8 g) was obtained by column chromatography using toluene/ethyl acetate as eluent. Yield 51% $^1H$ NMR ($CDCL_3$) δ ppm: 1.00 (d, 3H, -CH-$CH_3$-) 2.6 (m, 1H, CH-$CH_3$-), 1.4 (s, 9H, -OC$(CH_3)_3$) 4.4 (s, 2H, -CO$CH_2$O-) 6.25 (d, 2H, *J*=8.54), 7.2 (m, H *Trt*). Anal. ($C_{38}H_{43}N_3O_5S$) C, H, O, N, S. 1.1.2.8 Preparation of 12 by removal of Trt and BOC groups.

Example of 12a (Compound (IIIb) wherein $R_3=R_4=R_6=R_7=H$ and $R_{12}$ = -O$(CH_2)$m-CONHCONH$_2$).

A solution of 11a (0.8 g) in 15 mL of 1:1 TFA:$CH_2Cl_2$ (15 mL) was stirred at room temperature for 3h. The solvents were removed in vacuo, and the resulting oily residue was washed with hexane, dissolved in 1:1 methanol: water containing 0.1% TFA and purified by chromatography using toluene/ethyl acetate as eluent to give compound 12a 0.5 g. Yield 41% $^1H$ NMR ($CDCl_3$) δ ppm: 1.03 (d, 3H, J= 6.7 Hz), 2.3 (m, 1H, -$CH_2$CH$NH_2$-), 3.35 (m, 1H, -CHNHCO-) 3.75 (m, 1H, -NH CO $CH_2$-) 4.02 (s, 2H, -CO$CH_2$O-) 6.9 (d, 2H, J= 8.54 Hz).

Example 2

Preparation of immunogens

Immunogens are prepared from compounds II or IIIa, IIIb by coupling various numbers of compound molecules to a protein carrier such as tetanus toxin, cholera toxin B subunit, bovine serum albumin, ovalbumin, or KLH.

2.1 - Coupling of compounds having formula II.

Compounds having formula II were coupled to a protein carrier by the mixed anhydride method or by using other coupling agents.
The conjugation of compound (II) was achieved in 2 steps: Maleimide were introduced by acylation of the protein carrier. The cross linking was achieved as previously described (Yoshitake and al., J Biochem, Tokyo, 1982; 92: 1413-24)

Example 3

Preparation of murine monoclonal antibodies (mAbs)

3.1 - Immunization of mice

Five BALB/c mice were immunized by intraperitoneal injection of 50 μg of tetanus toxin (TT) conjugate to hapten 1 (TT-Met1). Injections were performed at three to six week intervals, according to the schedule shown in Table I below. The immunogen was mixed with complete or incomplete Freunds adjuvant at a 1:1 ratio (vol.:vol.). Injection 1 was performed in complete Freunds adjuvant (FC) whereas subsequent injections were performed in incomplete Freunds adjuvant (IFC). Blood samples were collected from animals before the first injection and three to five days after each injection.

Table I

| | Immunization Schedule |
|---|---|
| | Series TT-Met1 |
| Withdrawing T0 | |
| Injection 1:<br>50 μg FC, IP | Week 12 |

Table I (continued)

| Immunization Schedule | |
|---|---|
| | Series TT-Met1 |
| Withdrawing T0 | |
| Injection 2: 50 µg IFC, IP | Week 15 |
| Injection 3: 50 µg IFC, IP | Week 21 |
| Injection 4: 50 µg IFC, IP | Week 24 |

The immunological response of the animals was monitored by ELISA performed with Bovine serum albumin (BSA) conjugate (BSA-Met1). Briefly, 50 µL of BSA-Met1 at 10 µg/mL in 0.1 M carbonate buffer, pH 9.0, were applied to ELISA plates (Maxisorb, Nunc). After incubation for 1H at 37°C and/or 18H at 4°C, plates were saturated by addition of 200 µL of 1% gelatin dissolved in Tris saline buffer (TBS), pH 7.4, and incubation for 1 to 2H at 37°C. Fifty µL of serially diluted animal serum were incubated for 1H at 37°C before washing with TBS containing 0.05% Tween 20 and further incubation with peroxidase labeled secondary antibody (peroxidase labeled rabbit IgG directed to mouse IgG; BioAtlantic, Nantes, France). Immunocomplexes were revealed by OPD staining. Titers were determined as the inverse of the dilution giving an OD value immediately >0.2. Data obtained with 5 mice (M30 to M34) are presented in Table II below.

Table II

| Mouse Antibody Titers | | | | | |
|---|---|---|---|---|---|
| TT-Met1 | | | | | |
| | M30 | M31 | M32 | M33 | M34 |
| T1 | 4000 | 2000 | 8000 | 4000 | 4000 |
| T2 | 4000 | 1000 | 4000 | 2000 | 2000 |
| T3 | 8000 | 8000 | 16000 | 16000 | 16000 |

Strong immunological responses were obtained in all mice. Mouse #34 was selected for preparation of monoclonal antibodies in hybridoma cell culture.

3.2 - Fusion with myeloma cells.

Sp2/O-AG14 murine myeloma cells were used as fusion partner. Fusion was performed by the polyethylene glycol method essentially according to Loirat.[15]

3.3 - Selection of hybridomas

Hybridomas were initially selected by ELISA with plates coated with BSA-Met1. Hybridomas secreting antibodies that significantly bound to BSA-Met1 (OD>0.3) were amplified to produce 1 mL culture supernatants in 24 well culture plates. Supernatants were again tested by ELISA to determine titers. Selected antibodies were then tested for determination of isotype and investigated by competition ELISA using methamphetamine and ecstasy as inhibitors.

3.4 - Competition test

Methamphetamine and ecstasy were each dissolved at 1 mg/mL in ion free water (Milli Q quality) and tested at 0.1 and 0.01 mg/mL for inhibitory activity. The competition ELISA was performed as follows. Thirty µL of hybridoma supernatant was diluted in order to obtain an OD value in the range 0.7-1.0 (30 to 50% of maximum OD value) and incubated with 30 µL of inhibitor for 1H at 37°C. Fifty µL of the mixture were added to microwells of ELISA plates coated with BSA-Met1, incubated for 1H at 37°C. Plates were further treated as described in 3.1. Inhibitory activity of methamphetamine and ecstasy was evaluated in percentage [1-(OD with inhibitor/OD without inhibitor)x100].

3.5 - Isotype determination was performed as described (Loirat et al., 1992).[15]

3.6 - Cloning

Hybridomas secreting antibodies with expected characteristics, i.e. OD value >1.0, isotype: IgG1, IgG2a or IgG2b, inhibition >50% with methamphetamine and ecstasy at 0.01 mg/mL, were cloned by limiting dilution as described (Loirat et al., 1992). Hybridoma cells were diluted in culture medium and distributed in 96 well culture plates so that 1 or 0.5 cell was statistically distributed per well. Culture occurred for 8 to 12 days before screening as described in 3.3.
Cloning was performed twice. Thirty-five secreting hybridomas and six final clones (6DL2) were obtained.

3.7 - Characterization

Characterization was performed on culture supernatants produced in 50 mL culture flasks and on purified antibodies obtained from ascitic fluids. Ascites were produced in BALB/c mice : animals were pretreated with pristane for 14 days, injected intraperitoneally with about 3 million hybridoma cells and ascites were collected 8 to 12 days after cell injection. Purification of IgG was performed by affinity chromatography on immobilized protein A; bound IgGs were eluted from the gel at pH 6.0 (IgG1) or pH 3.0 (IgG2b).

Characterization involves :

- Isotype determination;
- Determination of titer by ELISA and RIA ;
- Determination of affinity and cross reactivity by RIA ;
- Inhibition test with a series of analogs (see Table III of results);
- Cross reactivity analysis with human blood cells and human plasma proteins.

3.7.1 - RIA method:

Sample Titer is the dilution inverse of antibodies, which binds 50% of labeled metabolite present.
Different antibody dilutions are incubated with a known quantity of tracer. After incubation, tracer-antibody complexes are precipitated and non-bound tracer quantity present in the supernatant is measured.
Affinity is determined by testing the cross-reactivity between labeled and unlabeled metabolites. It is calculated according the method of Müller.[16]
Different dilutions of unlabeled metabolite are incubated with known tracer and antibody concentration. After incubation, the metabolite-antibody complexes are precipitated and non-bound tracer quantity present in supernatant is measured.

Ka is calculated according by the following formula :

$$Ka = \frac{1}{(1\text{-}1{,}5b+0{,}5\ b^2)*(IC_{50}\text{-}T)}$$

wherein,

b = Ratio [(AT-NS)-free max]/(AT-NS), wherein (AT-NS) is the maximal of labelled metabolite bound to the antibody.
T : Total molar concentration of labelled metabolite.
$IC_{50}$ = Concentration of unlabeled metabolite which inhibits 50% of the binding between antibody and tracer.

Titers were determined with methamphetamine-$^3$H(at a concentration of $1.8^x\ 10^{-9}$M) and ecstasy-$^3$H (at a concentration of $7.6\ 10^{-10}$M). Affinity is calculated with methamphetamine oxalate.

3.7.2 Characteristics of selected murine monoclonal antibodies.

Two clones from fusion 243 performed at the Etablissement Français de Sang-Pays de la Loire (site de Nantes) were obtained after 2 limiting dilutions. They are identified as DASM243-6H5D1C4 and DASM243-3A10A6A2.

3.7.3 - Isotypes, titers and affinity constants were determined by RIA and ELISA with purified mAbs. The results are presented in Table III below:

Table III

| Characteristics of Selected Antibodies | | | | |
|---|---|---|---|---|
| Antibody | Isotype | Titer | IC50 (nmol/mL) | Ka(M-1) |
| DASM243-6H5D1C4 | IgG2b | 128 | 0.0144 | 2.58E+08 |
| DASM243-A10A6A2 | IgG1 | 2.27 | 0.468 | 5.58E+06 |

Cross reactivity with a series of methamphetamine analogs was determined by ELISA. The results are presented in Table IV below as % inhibition as compared with the antigen Met1.

Table IV

| Cross reactivity with analogs | | | |
|---|---|---|---|
| INHIBITOR | MW | 3A10A6A2 (%) | 6H5D1C4 (%) |
| Hapten 1 (Met1) | 193 | 100 | 100 |
| Hapten 2 (Met2) | 353 | 242 | 686 |
| 4-methoxymetamphetamine | 269 | 6 | 8 |
| 4-methoxyamphetamine | 255 | 79 | 16 |
| Ephedrine | 165 | 32 | 26 |
| Methamphetamine | 239 | 229 | 68 |
| Ecstasy | 229 | 209 | 6 |
| 4-methylthioamphetamine | 271 | 58 | 100 |
| N-ethylamphetamine | 255 | 54 | 72 |
| Nor-ephedrine | 188 | 2 | 1 |
| 3-hydroxytyramine | 190 | 2 | 1 |
| Epinephrine | 333 | 3 | 1 |
| 3-hydroxy-4-methoxyphenetylalamine | 204 | 2 | 1 |
| Methyl pseudo ephedrine | 179 | 38 | 2 |
| Amphetamine | 255 | 29 | 1 |

As shown in Table V both antibodies recognized methamphetamine and ecstasy. 6H5D1C4 recognized both methamphetamine and ecstasy but it recognized methamphetamine 10 times more than ecstasy.

3.7.4 Cross reactivity with human blood cells

Cross reactivity of the mice antibodies with human peripheral blood cells was investigated by flow cytometry. Fresh cells were used, collected from same-day blood specimens (or one day old for red and white cells, stored at 4°centigrade) taken from healthy persons who did not take drugs. The results are presented below. The data are presented as mean fluorescence intensity.

Table V

| Lack of cross-reactivity with human blood cells | | | | |
|---|---|---|---|---|
| Sample | Neg. control culture sup | Positive control (*) | 3A10A6A2 | 6H5D1C4 |
| Erythrocytes | 2.97 | 76.24 | 2.97 | 2.80 |
| Lymphocytes | 8.87 | 1302.93 | 9.52 | 9.28 |

* Positive controls: specific monoclonal antibodies directed to erythrocytes (anti-Glycophorin A), to white blood cells (anti-HLA Class I) and to platelets (anti-GPIIIa).

Table V (continued)

| Lack of cross-reactivity with human blood cells | | | | |
|---|---|---|---|---|
| Sample | Neg. control culture sup | Positive control (*) | 3A10A6A2 | 6H5D1C4 |
| Monocytes | 23.41 | 159.02 | 29.32 | 23.38 |
| Granulocytes | 35.83 | 346.49 | 20.83 | 19.38 |
| Platelets | 2.11 | 230.73 | 4.64 | 3.98 |

* Positive controls: specific monoclonal antibodies directed to erythrocytes (anti-Glycophorin A), to white blood cells (anti-HLA Class I) and to platelets (anti-GPIIIa).

These data demonstrate that the two monoclonal antibodies tested do not exhibit cross reactivity activity with human peripheral blood cells.

3.7.5 Cross reactivity with human plasma proteins.

Cross reactivity of the antibodies with human plasma proteins was investigated by western blotting as indicated in the legend of the Figure 4.

3.7.5.1. Experimental:

1) Electrophoresis of plasma proteins solubilized in SDS according to Laemmli (1970) in a 10% polyacrylamide gel ; 2) electrophoretic transfer to nitrocellulose sheet; 3) immunoblotting: membranes were saturated in 1% fat free milk, incubated with mAbs to methamphetamine (antibodies diluted 1/10) for 1H at RT, incubated with secondary antibodies (peroxidase labelled anti-mouse IgG), and detected using the ECL method (Amersham). The results are shown in Figure 4: Lanes 1 to 5: 1) 3A10A6A2; 2) 3A10E5E7; 3) antibody 6H5D1C4; 4) antibody 6H5E1G12; 5) culture medium (negative control).
The immunoblotting analysis demonstrates that there is no significant cross reactivity with human plasma proteins.

Example 4.

Vaccine preparation

Human vaccines comprising amphetamine-hapten conjugates conjugated to immunogens are prepared from compounds II or IIIa, IIIb by coupling the compound molecules to the B subunit of cholera toxin using the carbodiimide method described above. The preparations are dissolved in saline solution at a protein concentrations of 200 µg of protein per mL, filter sterilized and stored at 4° C.

Example 5.

Immunization protocols

Serum samples from human adult patients are taken immediately before immunization and approximately four weeks after immunization to determine efficacy of the vaccination protocol. The patients are divided into groups that receive conjugate vaccines prepared with different amphetamine derivatives. The groups are similar with respect to gender, race, and age at the first dose of conjugate vaccination. To determine the optimum dose of coupled amphetamine derivative: cholera B toxin subunit for eliciting an antibody response, human adults are challenged with a single intramuscular injection of the conjugate. The dose of conjugate ranges from 1 to 100 µg of protein in 0.5. mL of saline solution.

Example 6.

Evaluation of immune response

The purpose of this experiment is to evaluate conjugates prepared in as in Example 4 for their ability to induce an antibody response in humans. Anti-amphetamine or anti-amphetamine derivative antibody response also is useful as a measure of both short and long-term vaccine efficacy.
To determine antibody titer following vaccination with various protein:amphetamine derivatives, blood specimens from vaccinated adults are analyzed by ELISA. The blood specimens include samples from each patient before (i.e., a negative control) and about four weeks after vaccination. The ELISA is carried out by coating 96 wells polystyrene plates with 1 μg/well of amphetamine at 37° C. for 2 hrs. Non specific binding sites are blocked with 5% powdered milk in phosphate buffered saline (PBS) and 0.1% Tween 80 at 37° C. for 30 minutes. The plates are washed once with PBS and 0.1% Tween. Blood samples are tested at various dilutions (for example, 1:100, 1:1000 and 1:10000) by adding each dilution to the antigen-coated plates in triplicate wells. After 2 hrs of incubation, the plates are washed three times with PBS and 0.1% Tween. One hundred μl of horesradish peroxidase conjugated goat anti-human IgG (Boehringer-Mannheim Biochemicals), diluted 1 :250 to 1 :1000, are added per well. The plate is incubated at 37° C. for 1 hr and washed three times with PBS and 0.1% Tween. Fifty μl of a 1:50 dilution of o-phenyl-diamine in citrate buffer, pH 5.0, with 1 μl/mL of 30% $H_2O_2$ are added and incubated at room temperature for 20 minutes. The absorbance at 495 nm is measured in each well and results are expressed as mean optical density of triplicate wells.

Example 7

Active Immune Protection Against Amphetamine Toxicity

The ability of amphetamine conjugate preparations to protect against the toxic effects of amphetamine and derivatives is demonstrated in an animal model by determining the lethal dose of each compound (expressed as the $LD_{50}$) in immunized and non-immunized mouse populations. Mice are immunized with a single intramuscular injection of a conjugate preparation in a saline solution. The first injection may be followed by a booster injection of the same conjugate. After vaccination, each of the mice are bled and serum antibody titers determined by an ELISA similar to that in Example 6, substituting anti-mouse antibodies-enzyme conjugates for anti-human antibody conjugates. Optimum conjugate concentration within each vaccine preparation is determined experimentally by ELISA. Once a efficacious vaccine regimen is established experimentally for each conjugate preparation, mice are separated into groups and vaccinated with the different conjugate preparations. An unvaccinated control group receives a saline control. Within each group, the mice are challenged with methamphetamine or its derivatives at various concentrations to determine the $LD_{50}$ for each preparation. Efficacious vaccine regimens protect the animals from at least one amphetamine derivative by significantly increasing the $LD_{50}$ of at least one derivative within that group. Protection against multiple derivatives is also determined experimentally; following vaccination against a single amphetamine derivative, protection from the toxic effects of other derivatives is determined by observing a significant increase in the $LD_{50}$ after challenge with the different derivatives. The broadest protection, i.e., the active vaccines that provide protection against a wide range of amphetamine derivatives is used to identify candidate preparations for use in human clinical trials.

Example 8

Passive Immune Protection Against Amphetamine Toxicity

The ability of sera prepared from animals vaccinated with amphetamine conjugate preparations to protect against the toxic effects of amphetamine and derivatives is demonstrated in an mouse model. Rabbits are immunized with a vaccine derivative that provides broad protection against various amphetamine derivatives determined with the procedure of Example 7. Four weeks after immunization, the antibody titer is determined in the rabbits by the ELISA protocol of Example 6, substituting anti-rabbit antibodies-peroxidase conjugates for anti-human antibody peroxidase conjugates. The rabbits are bled and serum is separated from the whole blood, filter-sterilized and stored at 4° C. Mice are separated into groups and challenged with an $LD_{50}$ of one amphetamine per group. Immediately following challenge with amphetamine derivatives, the mice are administered rabbit serum by intravenous injection. The minimum protective volume required to increase significantly each $LD_{50}$ is determined experimentally. The efficacy of a protection protocol in immunized and non-immunized mouse populations after challenge with an amphetamine derivative is determined by a significant increase in the $LD_{50}$ of each tested amphetamine derivative in any of the groups of mice. The broadest protection, i.e., the passive immunization that provides protection against the widest range of amphetamine derivatives, is used to identify candidate preparations for passive protection of humans in clinical trials.

The above results show that antibodies specific for at least two amphetamines and/or amphetamine derivatives can be prepared using the compounds of the invention.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporate by reference.

The invention now having been fully described, it will be apparent to one of ordinary skill in the art that many changes and modifications can be made thereto without departing from the spirit or scope of the appended claims.

## Claims

**1.** An (S)-enantiomer compound having the formula (I):

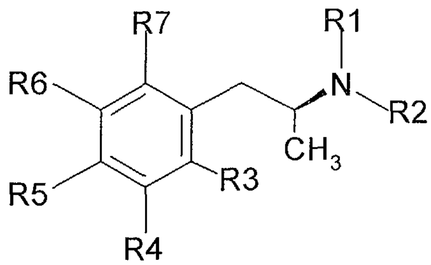

(I)

wherein:

$R_1$ and $R_3$ are selected from the group consisting of hydrogen, and a $C_1$-$C_3$ alkyl;
$R_2$ is selected from the group consisting of hydrogen, a $C_1$-$C_3$ alkyl and a polymethylene chain: $(CH_2)_n$-COOH wherein n is an integer between 1 and 6;
$R_4$, $R_6$ and $R_7$ aare the same or different and are selected from the group consisting of hydrogen, a halogen, -$OR_9$ and-$SR_9$, wherein $R_9$ is hydrogen or a $C_1$-$C_3$ alkyl; and
$R_5$ is selected from the group consisting of hydrogen, a polymethylene chain: -$(CH)_m$-$R_{10}$ and an oxy-polymethylene chain: -O-$(CH_2)_m$-$R_{10}$, wherein -$R_{10}$ is selected from the group consisting of carboxyl, thiol, -$CONHR_{13}SH$, and -$CONHCHR_{11}SH$, wherein $R_{13}$ is selected from the group consisting of-CH(COOH)$CH_2$- and -$(CH_2)_m$-, wherein m is an integer between 1 and 4, with the proviso that when $R_1$ is hydrogen and $R_2$ is a methyl or when $R_1$ is a methyl and $R_2$ is hydrogen, then $R_5$ is not a polymethylene chain: -$(CH_2)_m$-COOH.

**2.** A compound having formula (II)

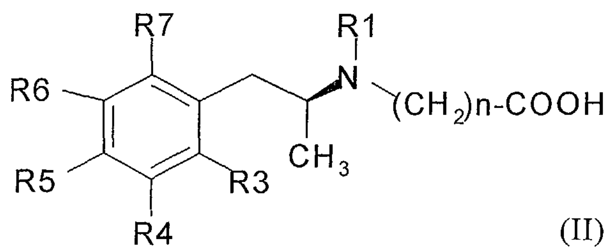

(II)

wherein

n is an integer between 1 and 6,
$R_1$ and $R_3$ are selected from the group consisting of hydrogen, and $C_1$-$C_3$ alkyl; $R_4$, $R_6$ and $R_7$ are the same or different and are selected from the group consisting of hydrogen, a halogen, -$OR_9$ and -$SR_9$, wherein $R_9$ is hydrogen or a $C_1$-$C_3$ alkyl; and
$R_5$ is selected from the group consisting of hydrogen, a polymethylene chain: -$(CH)_m$-$R_{10}$ and an oxy-polymethylene chain: -O-$(CH_2)_m$-$R_{10}$, wherein -$R_{10}$ is selected from the group consisting of carboxyl, thiol, -$CONHR_{13}SH$, and -$CONHCHR_{11}SH$, wherein $R_{13}$ is selected from the group consisting of-CH(COOH)$CH_2$- and -$(CH_2)_m$- and wherein m is an integer between 1 and 4, with the proviso that when $R_1$ is hydrogen and $R_2$ is a methyl or when $R_1$ is a methyl and $R_2$ is hydrogen, then $R_5$ is not a polymethylene chain: -$(CH_2)_m$-COOH.

**3.** The compound according to Claim 2, having formula (VI):

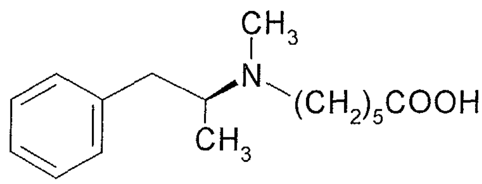

(VI).

**4.** The compound according to Claim 2, having formula (VII):

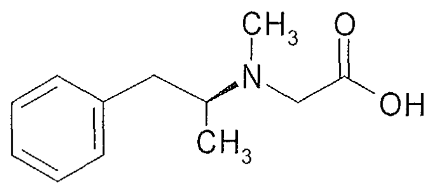

(VII).

**5.** A compound having formula (IIIa):

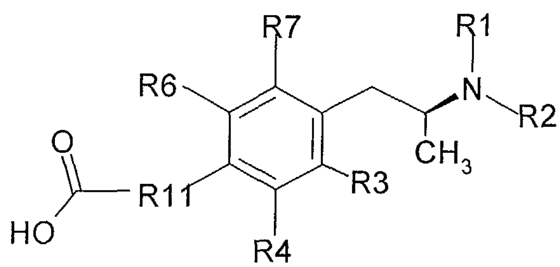

(IIIa)

wherein;

$R_1$ and $R_3$ are selected from the group consisting of hydrogen, and $C_1$-$C_3$ alkyl;
$R_2$ is selected from the group consisting of hydrogen, a $C_1$-$C_3$ alkyl and a polymethylene chain: $(CH_2)_n$-COOH, wherein n is an integer between 1 and 6;
$R_4$, $R_6$ and $R_7$ are the same or different and are selected from the group consisting of hydrogen, a halogen, -$OR_9$ and -$SR_9$, wherein $R_9$ is hydrogen or a $C_1$-$C_3$ alkyl; and,
$R_{11}$ is selected from the group consisting of a polymethylene chain: -$(CH_2)_m$- and an oxy-polymethylene chain: -$O(CH_2)_m$-, wherein m is an integer between 1 and 4, with the proviso that when $R_1$ is a methyl, then $R_{11}$ is not a polymethylene chain: -$(CH_2)_m$-.

**6.** The compound according to Claim 5 having formula (IV):

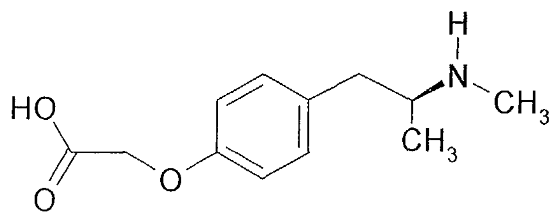

(IV).

**7.** A compound having formula (IIIb)

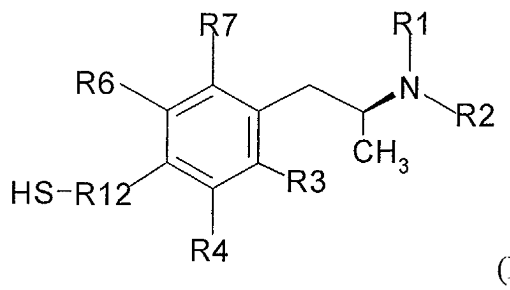

(IIIb)

wherein,
$R_1$ and $R_3$ are selected from the group consisting of hydrogen, and a $C_1$-$C_3$ alkyl;
$R_2$ is selected from the group consisting of hydrogen, a $C_1$-$C_3$ alkyl and a polymethylene chain: $(CH_2)_n$-COOH, wherein n is an integer between 1 and 6;
$R_4$, $R_6$ and $R_7$ are the same or different and are selected from the group consisting of hydrogen, a halogen, $-OR_9$ and $-SR_9$, wherein $R_9$ is hydrogen or a $C_1$-$C_3$ alkyl ; and
$R_{12}$ is selected from the group consisting of a polymethylene chain:
$(CH_2)_mCONHR_{13}$ and an oxy-polymethylene chain: $-O(CH_2)_mCONHCH-R_{13}$;
wherein $R_{13}$ is selected from the group consisting of-$CH(COOH)CH_2$- and $-(CH_2)_m$-, wherein m is an integer between 1 and 4, with the proviso that when $R_1$ is hydrogen and $R_2$ is a methyl or when $R_1$ is a methyl and $R_2$ is hydrogen, then $R_5$ is not a polymethylene chain: $-(CH_2)_m$-COOH.

**8.** The compound according to Claim7 having formula (V):

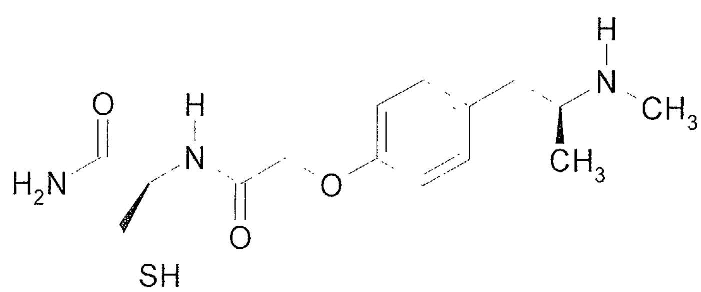

(V).

**9.** A method for obtaining a compound according to Claim 4, said method comprising the steps of:

(a) alkylating a methamphetamine derivative having formula (IX) as shown in Figure 1 by addition of Br $(CH_2)_nCOOBn$, wherein n is an integer between 1 and 6, whereby an addition product of formula (X) as shown in Figure 1 is obtained ; and
(b) hydrogenating said addition product of formula (X), whereby a compound according to Claim 4 is obtained.

**10.** A method for obtaining a compound according to Claim 5, said method comprising the steps of:

(a) condensing a a compound of formula (XI) as shown in Figure 2, with $CH_3CH_2\text{-}NO_{2,}$ whereby a nitrostyrene derivative of formula (XII) as shown in Figure 2 is obtained;
(b) reducing said nitrostyrene derivative (XII), whereby an amphetamine derivative of formula (XIII) as shown in Figure 2 is obtained;
(c) alkylating the primary amine of said amphetamine derivative whereby a compound of formula (XIV) as shown in Figure 2 is obtained;
(d) acylating the amine group on said amphetamine derivative (XIII) or said compound of formula (XIV), whereby a protected derivative of a compound having formula (XV) as shown in Figure 2 is obtained;
(e) hydrogenating said protected derivative (XV), whereby a compound having a formula (XVI) as shown in Figure 2 is obtained;
(f) alkylating the phenol groups of said compound of formula (XVI) with benzylbromoalkylcarboxylate, whereby a benzylester compound of formula (XVII) as shown in Figure 2 is obtained;
(g) hydrogenating said benzylester compound of formula (XVII) whereby a carboxylic derivative of formula (XVIII) as shown in Figure 2 is obtained;
(h) deprotecting the amino group of said carboxylic acid derviative (XVIII); and
(i) separating the enantiomers so obtained, whereby compounds according to Claim 5 are obtained.

**11.** The method according to Claim 10 steps (a) through (g) further comprising the steps of:

(h) acylating (R)H-cys(Otrt)-NH2 with said carboxylic derivative (XVIII);
(i) separating the resulting diastereoisomer mixture whereby an S-isomer having formula (XIX) as shown in Figure 2 is obtained; and
(j) removing acid-sensitive protecting groups from said S-isomer having formula (XIX), whereby a compound according to Claim 7 is obtained.

**12.** The method according to Claim 10 or Claim 11, wherein said alkylating step (c) comprises:

contacting said compound of formula (XIII) with trifluoroacetic anhydride; alkylating the resulting trifluoroacetamide with an alkyl halide; and removing the trifluoroacetyl group in basic medium.

**13.** The method according to Claim 10 or Claim 11, wherein said alkylating step (c) comprises:

contacting said compound of formula (XIII) with an acid anhydride to obtain an amide; and
reducing said amide with lithium aluminium hydride.

**14.** A method for obtaining a compound according to Claim 5, said method comprising the steps of:

(a) reducing a tyrosine ester derivative of a compound of formula (XXII) as shown in Figure 3, whereby an alcohol derivative of formula (XXIII) as shown in Figure 3 is obtained;
(b) alkylating said alcohol derivative (XXIII) whereby a compound of formula (XXIV) as shown in Figure 3 is obtained;
(c) hydrogenating said compound of formula (XXIV) whereby a phenol derivative of formula (XXV) as shown in Figure 3 is obtained;
(d) contacting said phenyl derivative (XXV) with benzylbromoalkylcarboxylate whereby an ester derivative of formula (XXVI) as shown in Figure 3 is obtained; and
(e) hydrogenating said ester derivative (XXVI) whereby a compound according to Claim 5 is obtained.

**15.** An amphetamine-related immunogen comprising:

a compound having formula (I) covalently conjugated to a carrier.

**16.** An amphetamine-related immunogen I1 comprising:

a compound having formula (II) covalently conjugated to a carrier,

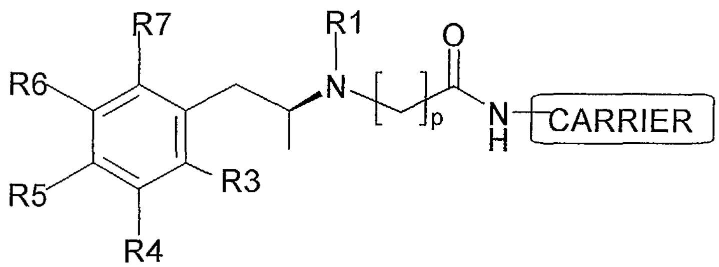

I1

wherein p is an integer between 1 and 6;
wherein $R_1$ and $R_3$ are selected from the group consisting of hydrogen, and $C_1$-$C_3$ alkyl;
$R_4$, $R_6$ and $R_7$ are the same or different and are selected from the group consisting of hydrogen, a halogen, $-OR_9$ and $-SR_9$, wherein $R_9$ is hydrogen or a $C_1$-$C_3$ alkyl; and
$R_5$ is selected from the group consisting of hydrogen, a polymethylene chain: $-(CH)_m-R_{10}$ and an oxy-polymethylene chain : $-O-(CH_2)_m-R_{10}$, wherein $-R_{10}$ is selected from the group consisting of carboxyl, thiol, $-CONHR_{13}SH$, and $-CONHCHR_{11}SH$, wherein $R_{13}$ is selected from the group consisting of-$CH(COOH)CH_2$- and $-(CH_2)_m-$ and wherein m is an integer between 1 and 4, with the proviso that when $R_1$ is hydrogen and $R_2$ is a methyl or when $R_1$ is a methyl and $R_2$ is hydrogen, then $R_5$ is not a polymethylene chain: $-(CH_2)_m-COOH$.

**17.** An amphetamine-related immunogen I2 comprising:

a compound having formula (IIIa) covalently conjugated to a carrier,

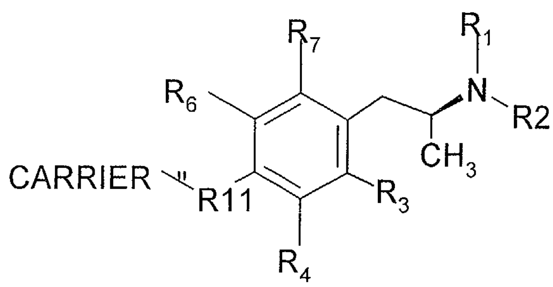

I2

wherein $R_1$ and $R_3$ are selected from the group consisting of hydrogen, and $C_1$-$C_3$ alkyl;
$R_2$ is selected from the group consisting of hydrogen, a $C_1$-$C_3$ alkyl and a polymethylene chain: $(CH_2)_n-COOH$, wherein n is an integer between 1 and 6; $R_4$, $R_6$ and $R_7$ are the same or different and are selected from the group consisting of hydrogen, a halogen, $-OR_9$ and $-SR_9$, wherein $R_9$ is hydrogen or a $C_1$-$C_3$ alkyl; and,
$R_{11}$ is selected from the group consisting of a polymethylene chain: $-(CH_2)_m-$ and an oxy-polymethylene chain: $-O(CH_2)_m-$, wherein m is an integer between 1 and 4, with the proviso that when $R_1$ is a methyl, then $R_{11}$ is not a polymethylene chain: $-(CH_2)_m-$.

**18.** An amphetamine-related immunogen I3 comprising:

a compound having formula (IIIb) covalently conjugated to a carrier,

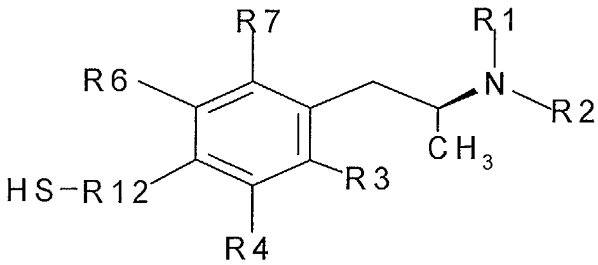

I3

wherein $R_1$ and $R_3$ are selected from the group consisting of hydrogen, and a $C_1$-$C_3$ alkyl;
$R_2$ is selected from the group consisting of hydrogen, a $C_1$-$C_3$ alkyl and a polymethylene chain: $(CH_2)_n$-COOH, wherein n is an integer between 1 and 6;
$R_4$, $R_6$ and $R_7$ are the same or different and are selected from the group consisting of hydrogen, a halogen, -$OR_9$ and -$SR_9$, wherein $R_9$ is hydrogen or a $C_1$-$C_3$ alkyl ; and
$R_{12}$ is selected from the group consisting of a polymethylene chain: $(CH_2)_mCONHR_{13}$ and an oxy-polymethylene chain: -$O(CH_2)_mCONHCH$-$R_{13}$;
wherein $R_{13}$ is selected from the group consisting of-$CH(COOH)CH_2$- and -$(CH_2)_m$-, wherein m is an integer between 1 and 4, with the proviso that when $R_1$ is hydrogen and $R_2$ is a methyl or when $R_1$ is a methyl and $R_2$ is hydrogen, then $R_5$ is not a polymethylene chain: -$(CH_2)_m$.

**19.** An amphetamine-related immunogen according to any one of Claims 15 to 18, wherein said carrier is a protein selected from the group consisting of cholera toxin, tetanus toxin, bovine serum albumin, ovalbumin, KLH, or a peptidic fragment of any of the proteins in said group.

**20.** The amphetamine-related immunogen TT-Met1 having the formula.

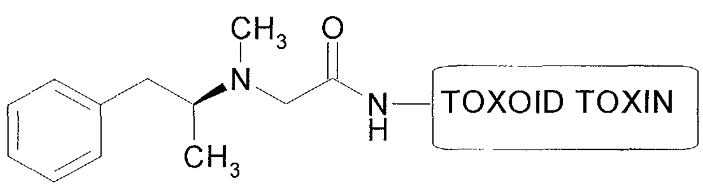

**21.** A pharmaceutical composition comprising:

at least one amphetamine-related immunogen according to Claim 15.

**22.** A vaccine comprising at least one amphetamine-related immunogens according to Claim 18 or Claim 20.

**23.** Antibodies or fragments or chains of immunoglobulins having affinity for one or more amphetamine or derivative of amphetamine .

**24.** The antibodies according to Claim 23, wherein said antibodies are monoclonal antibodies.

**25.** The antibodies according to Claim 23 or Claim 24, wherein said antibodies are neutralizing antibodies.

**26.** The antibodies according to Claim 24, wherein said antibodies are capable of binding to at least two different amphetamines or amphetamines derivatives..

**27.** The antibodies according to Claim 26, wherein said at least two different amphetamines or amphetamine derivatives comprise methamphetamine and ecstasy.

**28.** A monoclonal antibody produced by hybridoma DASM243-6H5D1C4.

**29.** A monoclonal antibody produced by hybridoma DASM243-3A10A6A2.

**30.** A composition comprising:

a cell line producing a monoclonal antibody according to Claim 24.

**31.** The composition according to Claim 30, wherein said antibodies are murine antibodies.

**32.** The murine hybridoma DASM243-645D1C4 having accession number CNCM 1-2750.

**33.** A cell line which produces monoclonal antibodies having the same antigenic specificity as that of monoclonal antibodies expressed by cell line DASM243-645D1C4.

**34.** Monoclonal antibodies derived from a hybridoma according to Claim 33.

**35.** An immunoglobulin fragment or chain derived from a monoclonal antibody having the binding specificity of a monoclonal antibody according to Claim 34.

**36.** Monoclonal antibodies according to Claim 24, labelled with an agent capable of providing a detectable signal.

**37.** An immunoglobulin fragment or chain according to Claim 35, labelled with an agent capable of providing a detectable signal.

**38.** A pharmaceutical composition comprising:

at least one antibody according to Claim 23, capable of binding to one or more amphetamine derivatives.

**39.** A compound according to any one of Claims 2 to 8, wherein said compound is the S-enantiomer.

**40.** Isolated nucleic acid encoding the heavy and light chains of monoclonal antibody DASM243-645D1C4 or DASM243-3A10A6A2.

**41.** Isolated nucleic acid encoding a polypeptide derived from monoclonal antibody DASM243-645D1C4 or DASM243-3A10A6A2.

**42.** The isolated nucleic acid according to Claim 44, wherein said polypeptide is a light chain protein.

**43.** The isolated nucleic acid according to Claim 43, wherein said nucleic acid is DNA.

**44.** The isolated nucleic acid according to Claim 43, wherein
said nucleic acid molecule is cDNA.

**45.** An expression vector comprising:

nucleic acid according to Claim 43 operably linked to transcriptional and translational regulatory regions.

A host cell comprising:

an expression vector according to Claim 48.

**46.** The host cell according to Claim 49, wherein said cell is a eukaryotic cell.

**47.** A process for producing an immunoglobulin specific for at least two amphetamines or amphetamine derivatives

or an immunologically functional immunoglobulin fragment thereof comprising at least the variable domains of the heavy and light chains of said immunoglobulin, said method comprising:

growing a host cell comprising expression vectors comprising nucleic acid encoding respectively the heavy and light chains of monoclonal antibody DASM243-645D1C4 or DASM243-3A1 or functional fragments thereof, so that said nucleic acid is expressed, whereby said immunoglobulin is obtained.

Figure 1
Synthesis compounds (II)

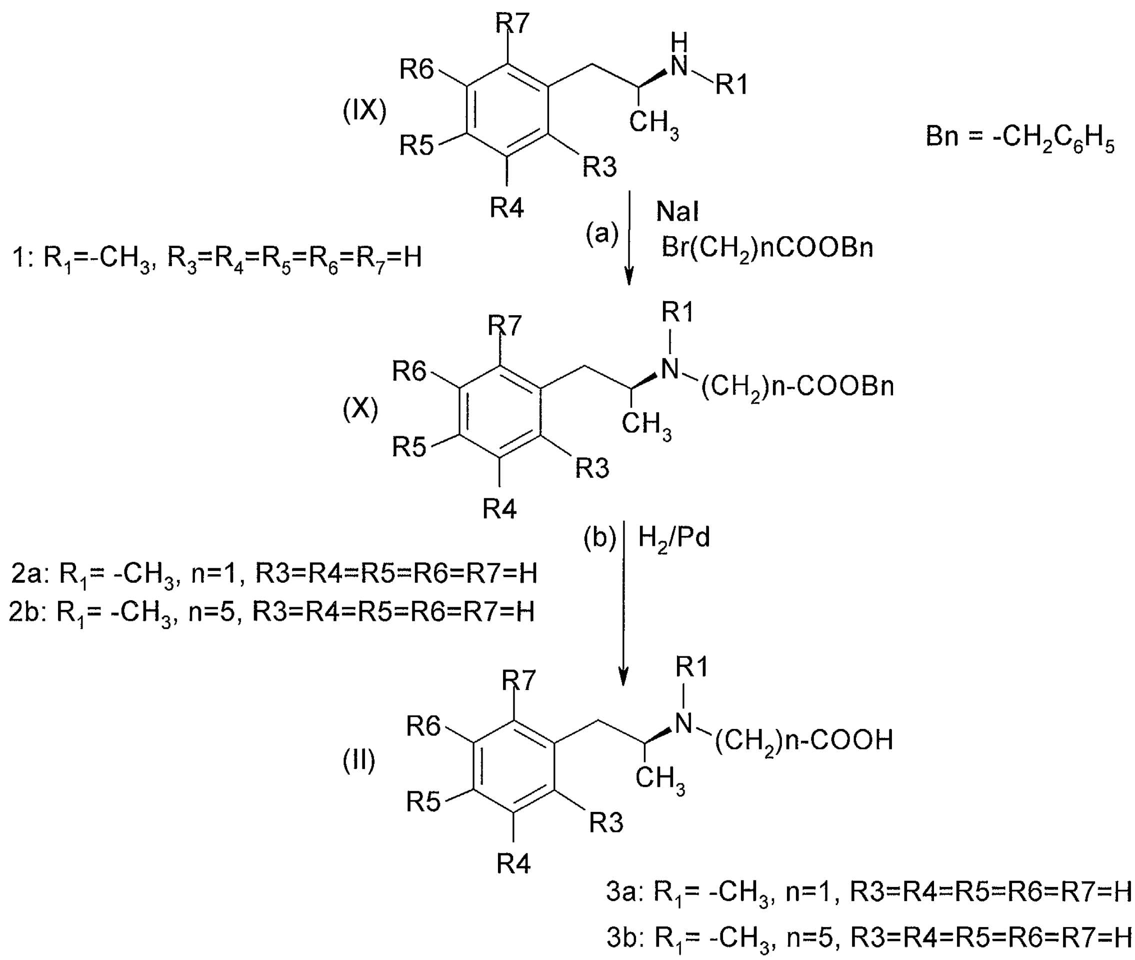

Figure 2

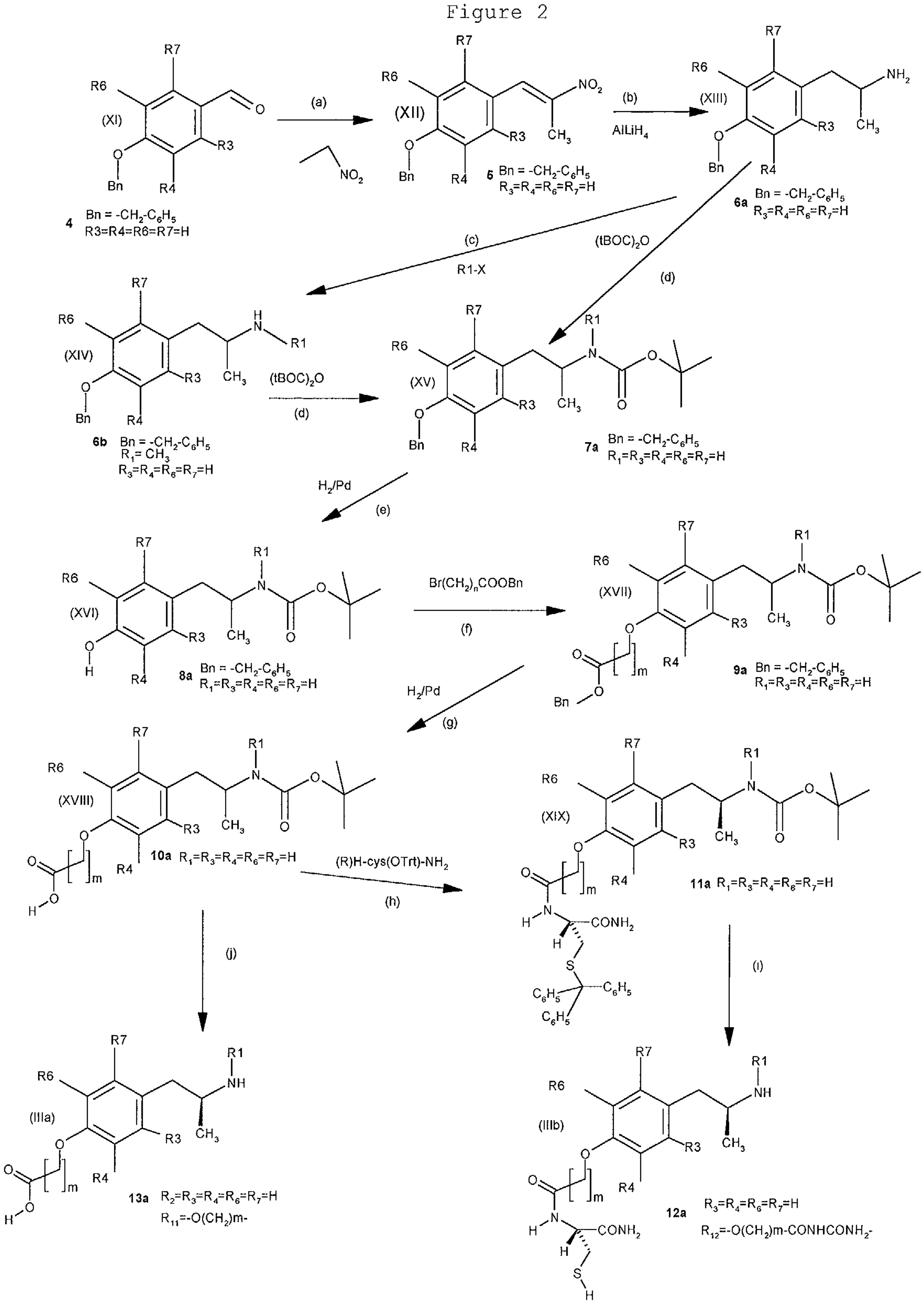
(a)
(b)
AlLiH4
4 Bn = -CH2-C6H5 R3=R4=R6=R7=H
5 Bn = -CH2-C6H5 R3=R4=R6=R7=H
6a Bn = -CH2-C6H5 R3=R4=R6=R7=H
(c)
R1-X
(tBOC)2O
(d)
6b Bn = -CH2-C6H5 R1= CH3 R3=R4=R6=R7=H
7a Bn = -CH2-C6H5 R1=R3=R4=R6=R7=H
H2/Pd
(e)
Br(CH2)nCOOBn
(f)
8a Bn = -CH2-C6H5 R1=R3=R4=R6=R7=H
9a Bn = -CH2-C6H5 R1=R3=R4=R6=R7=H
H2/Pd
(g)
10a R1=R3=R4=R6=R7=H
(R)H-cys(OTrt)-NH2
(h)
11a R1=R3=R4=R6=R7=H
(j)
(i)
13a R2=R3=R4=R6=R7=H
R11=-O(CH2)m-
12a R3=R4=R6=R7=H
R12=-O(CH2)m-CONHCONH2-

Figure 3

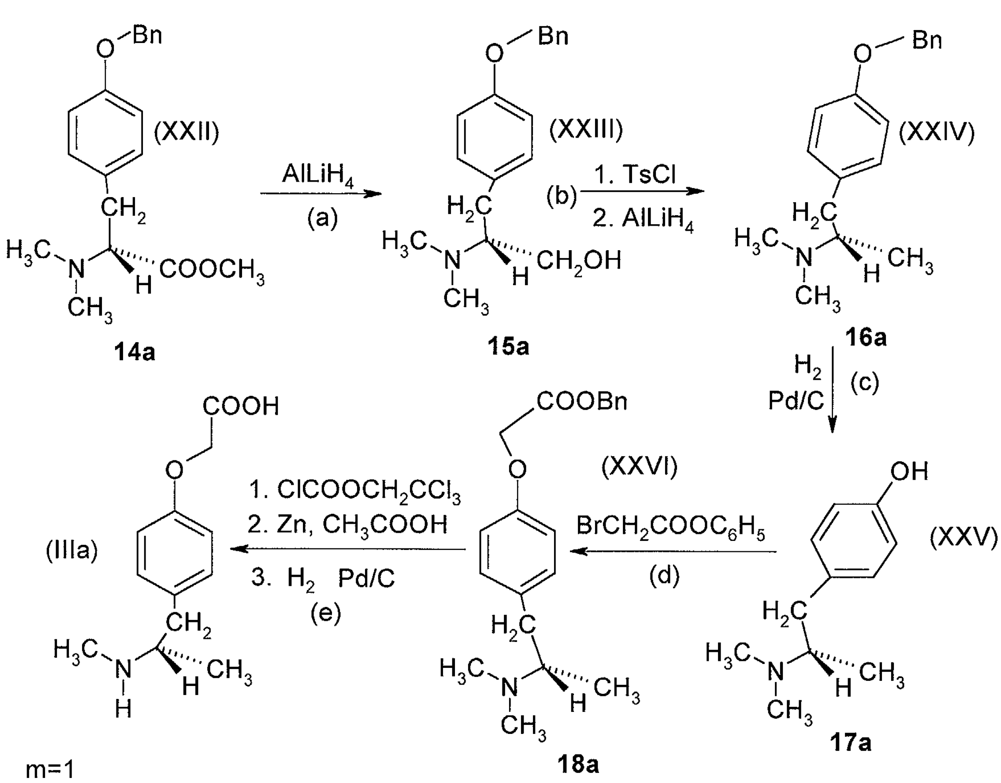


13a m=1
$R_3=R_4=R_6=R_7=H$
$R_1= -CH_3$

Figure 4

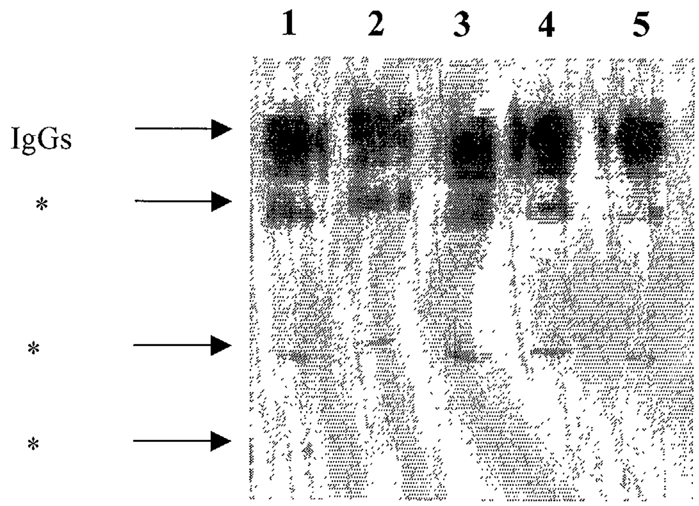


* degradation products from IgGs

Lines 1 to 5:
1) 3A10A6A2;
2) 3A10E5E7;
3) 6H5D1C4;
4) 6H5E1G12;
5) culture medium (negative control).

European Patent Office

**PARTIAL EUROPEAN SEARCH REPORT**

which under Rule 45 of the European Patent Convention shall be considered, for the purposes of subsequent proceedings, as the European search report

Application Number
EP 02 29 0169

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01 81424 A (TRUSTEES OF THE UNIVERSITY OF) 1 November 2001 (2001-11-01)<br>* page 1, line 15 - line 24 *<br>* page 4, line 15 - page 5, line 27 *<br>* page 6, line 23 - line 28 *<br>* page 13, line 27 - page 14, line 19 *<br>* page 18; example 2 *<br>* claims 1--13 *<br>* figure 4A *<br>--- | 1-47 | C07C229/14<br>C07C217/60<br>C07C323/60<br>C07K16/44<br>A61K39/00<br>A61K39/385<br>A61K39/395<br>C12N5/20<br>C12N5/10<br>C12N15/79 |
| X | PATENT ABSTRACTS OF JAPAN<br>vol. 014, no. 250 (C-0723),<br>29 May 1990 (1990-05-29)<br>& JP 02 069196 A (MATSUSHITA ELECTRIC IND CO LTD), 8 March 1990 (1990-03-08)<br>* abstract *<br>--- | 1-47 | |
| X | US 5 135 863 A (HSU CHEN-JUNG ET AL)<br>4 August 1992 (1992-08-04)<br>* column 3, line 22 - column 4, line 56 * | 23-47 | |
| X | * column 15, line 23 - line 66 *<br>---<br>-/-- | 7 | TECHNICAL FIELDS SEARCHED (Int.Cl.7)<br>C07C<br>C07K<br>C12N<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 11 September 2002 | O'Sullivan, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 02 29 0169

Claim(s) searched completely:
1-22, 28,29,32-35,37,39-41,47

Claim(s) searched incompletely:
23-27,30,31,36,38,42-46

Reason for the limitation of the search:

Present claims23-27,30,31,36,38,42-46 relate to antibodies/fragments/chains of immunoglobulins defined by reference to a desirable characteristic or property, namely having affinity for one or more amphetamine or derivative or amphetamine.

The claims cover all antibodies/fragments/chains of immunoglobulins having this characteristic or property, whereas the application provides support within the meaning of Article 84 EPC and/or disclosure within the meaning of Article 83 EPC for only a very limited number of such variables. In the present case, the claims so lack support, and the application so lacks disclosure, that a meaningful search over the whole of the claimed scope is impossible. Independent of the above reasoning, the claims also lack clarity (Article 84 EPC). An attempt is made to define the abovementioned by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the those antibodies/fragments/chains of immunoglobulin using the hapten compounds of the invention (claims 1-8) conjugated to the corresponding claimed carriers.

European Patent Office

# PARTIAL EUROPEAN SEARCH REPORT

Application Number
EP 02 29 0169

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 329 281 A (CHRISTENSON JAMES G ET AL) 11 May 1982 (1982-05-11)<br>* column 2, line 5 - column 3, line 18 *<br>* example 14 *<br>--- | 1-47 | |
| A | WO 97 49732 A (BIZZINI BERNARD ;DOX AL ITALIA SPA (IT); VOLPATO IVO (IT); GRABITZ) 31 December 1997 (1997-12-31)<br>* the whole document *<br>* page 6, line 18 *<br>--- | 1-47 | |
| A | US 5 976 812 A (HUBER ERASMUS ET AL) 2 November 1999 (1999-11-02)<br>* column 2, line 15 - line 47 *<br>* column 3, line 27 - line 66 *<br>--- | 1-47 | TECHNICAL FIELDS SEARCHED (Int.Cl.7) |
| A | US 5 470 997 A (BUECHLER KENNETH F ET AL) 28 November 1995 (1995-11-28)<br>* figure 1 *<br>* column 1, line 46 - line 54 *<br>* column 2, line 40 - column 3, line 2 *<br>* column 5, line 45 - column 7, line 50 *<br>* claim 1 *<br>--- | 1-47 | |
| X | DATABASE CROSSFIRE BEILSTEIN 'Online! Beilstein Institut zur Förderung der Chemischen Wissenschaften, Frankfurt am Main, DE;<br>Database accession no. 5015975<br>XP002213153<br>* abstract *<br>& KWIATKOWSKI, S ET AL: SYNTHESIS., vol. 12, 1989, pages 946-949, GEORG THIEME VERLAG. STUTTGART., DE ISSN: 0039-7881<br>---<br>-/-- | 1,2 | |

EPO FORM 1503 03.82 (P04C10)

European Patent Office

# PARTIAL EUROPEAN SEARCH REPORT

Application Number
EP 02 29 0169

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE CROSSFIRE BEILSTEIN 'Online! Beilstein Institut zur Förderung der Chemischen Wissenschaften, Frankfurt am Main, DE; Database accession no. 2111561 XP002213154 * abstract * & KWIATKOWSKI, S ET AL: SYNTHESIS., vol. 12, - 1989 pages 946-949, GEORG THIEME VERLAG. STUTTGART., DE ISSN: 0039-7881 --- | 1 | |
| X | DATABASE CROSSFIRE BEILSTEIN 'Online! Beilstein Institut zur Förderung der Chemischen Wissenschaften, Frankfurt am Main, DE; Database accession no. 2843137 XP002213155 * abstract * & INAYAMA ET AL: CHEMICAL AND PHARMACEUTICAL BULLETIN., vol. 25, - 1977 page 838 PHARMACEUTICAL SOCIETY OF JAPAN. TOKYO., JP ISSN: 0009-2363 --- | 1,2,4 | TECHNICAL FIELDS SEARCHED (Int.Cl.7) |
| X | DATABASE CROSSFIRE BEILSTEIN 'Online! Beilstein Institut zur Förderung der Chemischen Wissenschaften, Frankfurt am Main, DE; Database accession no. 7811559 XP002213156 * abstract * & PEINHARDT, G: PHARMAZIE., vol. 52, no. 12, - 1997 pages 937-939, VEB VERLAG VOLK UND GESUNDHEIT. BERLIN., DD ISSN: 0031-7144 --- | 1,5,6 | |

-/--

EPO FORM 1503 03.82 (P04C10)

European Patent Office

# PARTIAL EUROPEAN SEARCH REPORT

Application Number
EP 02 29 0169

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | O'NEILL M J ET AL: "The Merck Index - Thirteenth Edition" , MERCK INDEX. AN ENCYCLOPEDIA OF CHEMICALS, DRUGS, AND BIOLOGICALS, WHITEHOUSE STATION, NJ: MERCK & CO, US, PAGE(S) 1257 XP002213152<br>* page 97, "amphetamine" *<br>----- | 1 | |
| | | | TECHNICAL FIELDS SEARCHED (Int.Cl.7) |

EPO FORM 1503 03.82 (P04C10)

## ANNEX TO THE EUROPEAN SEARCH REPORT ON EUROPEAN PATENT APPLICATION NO. EP 02 29 0169

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2002

| Patent document cited in search report | Publication date | Patent family member(s) | Publication date |
|---|---|---|---|
| WO 0181424 A | 01-11-2001 | AU 5553701 A | 07-11-2001 |
| | | WO 0181424 A1 | 01-11-2001 |
| | | US 2001051158 A1 | 13-12-2001 |
| JP 02069196 A | 08-03-1990 | JP 2600325 B2 | 16-04-1997 |
| US 5135863 A | 04-08-1992 | AT 140082 T | 15-07-1996 |
| | | CA 1333890 A1 | 10-01-1995 |
| | | DE 68926779 D1 | 08-08-1996 |
| | | DE 68926779 T2 | 14-11-1996 |
| | | EP 0375422 A2 | 27-06-1990 |
| | | ES 2091202 T3 | 01-11-1996 |
| | | JP 2231467 A | 13-09-1990 |
| | | JP 2902699 B2 | 07-06-1999 |
| | | US 5328828 A | 12-07-1994 |
| US 4329281 A | 11-05-1982 | NONE | |
| WO 9749732 A | 31-12-1997 | IT MI961309 A1 | 29-12-1997 |
| | | AU 3437497 A | 14-01-1998 |
| | | WO 9749732 A1 | 31-12-1997 |
| | | EP 0934339 A1 | 11-08-1999 |
| US 5976812 A | 02-11-1999 | DE 19630102 A1 | 29-01-1998 |
| | | DE 59706730 D1 | 02-05-2002 |
| | | EP 0820984 A1 | 28-01-1998 |
| US 5470997 A | 28-11-1995 | AU 3940493 A | 08-11-1993 |
| | | CA 2132340 A1 | 14-10-1993 |
| | | EP 0634999 A1 | 25-01-1995 |
| | | JP 7505631 T | 22-06-1995 |
| | | WO 9320048 A1 | 14-10-1993 |

EPO FORM P0459

For more details about this annex : see Official Journal of the European Patent Office, No. 12/82